(19) **Europäisches Patentamt / European Patent Office / Office européen des brevets**

(11) **EP 4 169 021 B1**

(12) # EUROPEAN PATENT SPECIFICATION

(45) Date of publication and mention
of the grant of the patent:
**20.05.2026 Bulletin 2026/21**

(21) Application number: **21739872.6**

(22) Date of filing: **16.06.2021**

(51) International Patent Classification (IPC):
**G16B 5/30** $^{(2019.01)}$ **G16B 5/00** $^{(2019.01)}$

(52) Cooperative Patent Classification (CPC):
**G16B 5/00; G16B 5/30**

(86) International application number:
**PCT/US2021/037704**

(87) International publication number:
**WO 2022/026072 (03.02.2022 Gazette 2022/05)**

(54) **KINEMATIC MODELING OF BIOCHEMICAL PATHWAYS**

KINEMATISCHE MODELLIERUNG VON BIOCHEMISCHEN PFADEN

MODÉLISATION CINÉMATIQUE DE VOIES BIOCHIMIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.07.2020 US 202016942222**

(43) Date of publication of application:
**26.04.2023 Bulletin 2023/17**

(73) Proprietor: **X Development LLC
Mountain View, CA 94043 (US)**

(72) Inventors:
• **RUSSO, Frank
Mountain View, CA 94043 (US)**
• **HOENSELAAR, Andreas
Mountain View, CA 94043 (US)**

(74) Representative: **Trichard, Louis
Venner Shipley LLP
200 Aldersgate
London EC1A 4HD (GB)**

(56) References cited:
**US-A1- 2022 036 975 US-A1- 2022 148 684**

• **DEMIREL YASAR: "Nonequilibrium thermodynamics modeling of coupled biochemical cycles in living cells", JOURNAL OF NON-NEWTONIAN FLUID MECHANICS, 1 January 2010 (2010-01-01), pages 953 - 972, XP055837230, Retrieved from the Internet <URL:https://digitalcommons.unl.edu/cgi/viewcontent.cgi?article=1006&context=cbmedemirel> [retrieved on 20210902], DOI: 10.1016/j.jnnfm.2010.02.006**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD

The present disclosure relates to kinematic modeling of biochemical pathways, and in particular to general and scalable techniques for modeling *in silico* the kinetics of systems of connected biochemical reactions.

BACKGROUND

A biological system such as a living cell, or a population of living cells, can be modeled *in silico* such that the response of the living cell(s) to a variety of experiments can be performed quickly and cheaply in simulation. Demirel Yasar: "Nonequilibrium thermodynamics modeling of coupled biochemical cycles in living cells", Journal of non-Newtonian fluid mechanics, 1 January 2010, pages 953-972, XP055837230, is a review that describes component biology. This review focuses on the modeling of enzyme kinetics and fluctuation of single biomolecules acting as molecular motors, while in systems biology it focuses on modeling biochemical cycles and networks in which all the components of a biological system interact functionally over time and space. Biochemical cycles emerge from collective and functional efforts to devise a cyclic flow of optimal energy degradation rate, which can only be described by nonequilibrium thermodynamics. Therefore, this review emphasizes the role of nonequilibrium thermodynamics through the formulations of thermodynamically coupled biochemical cycles, entropy production, fluctuation theorems, bioenergetics, and reaction-diffusion systems. Fluctuation theorems relate the forward and backward dynamical randomness of the trajectories or paths, bridge the microscopic and macroscopic domains, and link the time-reversible and irreversible descriptions of biological systems. However, many of these approaches are in their early stages of their development and no single computational or experimental technique is able to span all the relevant and necessary spatial and temporal scales.

Simulated experiments may be performed to develop an understanding of the interrelationships of various environmental and other factors on the development of the biological system and/or on the biological system's effect on its environment. For example, simulated experiments may be performed to determine a set of environmental conditions that increases a growth rate of the biological system or that increases a rate of production of a substance of interest (e.g., an antibody, a hormone, a protein, an enzyme) by the biological system. Additionally or alternatively, the simulated experiments may be performed to develop an understanding of the effect of changes in the composition of the biological system (e.g., changes in the genetic or epigenetic makeup of the biological system) on the development or behavior of the biological system and/or to develop an understanding of the effect on changes in protein structure on the functionality of such proteins. For example, simulated experiments may be performed to determine a change in the genome of the biological system that increases a growth rate of the biological system and/or that increases a rate of production of a substance of interest by the biological system.

Biological systems and the biochemical processes thereof are typically modeled using various equations or functions and include many parameters, each corresponding to (e.g., 'modeling') an aspect of the structure and/or function of the biological system or biochemical process. Of the biochemical processes that need to be modelled to both infer molecular mechanisms and predict biological responses, many are enzyme reactions typically described using a system of differential equations. Modelling enzyme reactions and enzymatic cascade networks, however, often requires the simulation of multiple associated reactions. This inevitably increases the complexity of the system of differential equations, and exponentially increases the number of free kinetic parameters required for the modeling. The increase in free kinetic parameters can make it difficult to constrain all parameters simultaneously using a limited amount of available experimental data and can result in the derivation of biochemical process models with limited predictive power. Accordingly, there is a need for improving upon the modeling of biochemical processes with generalizable and scalable techniques to reduce parameter dimensionality without reducing the topological complexity required to capture key kinetic features in the biochemical processes.

SUMMARY

The invention is defined by the appended claims. It comprises a computer-implemented method as set forth in claim 8.

In some embodiments, the prediction model comprises a loss function constructed to measure a difference between the in silico behavior of the reaction and the expected behavior of the reaction, and where the difference is measured based on a comparison between a maximum velocity Vmax and a Michaelis constant Km of the in silico behavior of the reaction to the maximum velocity Vmax and the Michaelis constant Km of the expected behavior of the reaction.

In some embodiments, the plurality of component steps comprise at least two binding component steps and at least one transition component step, where each binding component step of the at least two binding component steps comprises: (i) binding between an enzyme and a substrate, or (ii) dissociation of an enzyme from a product, and where each transition component step of the at least one transition component step comprises a chemical conversion of an enzyme: substrate complex to a product:enzyme complex.

In some embodiments, the binding between the substrate and the enzyme contributes a first $\Delta G$ overall energy to the energy profile, the dissociation of the enzyme from the product contributes a second $\Delta G$ overall energy to the energy profile, and the chemical conversion of the enzyme: substrate complex to the product:enzyme complex contributes a $\Delta G\ddagger$ activation energy and a third $\Delta G$ overall energy to the energy profile.

In some embodiments, each forward rate equation has a form of a forward rate constant kon or kfwd multiplied by a single concentration or two concentrations, and where each reverse rate equation has a form of a reverse rate constant koff or krev multiplied by a single concentration or two concentrations.

In some embodiments, the deriving the in silico behavior of the reaction comprises numerically integrating across the standard mathematical constructs using a system of ordinary differential equations to determine the contribution of each component step to the rate of change of molecules within the reaction.

In some embodiments, the system of ordinary differential equations is represented by a single matrix built piecewise indicating the contribution of each component step to the rate of change of the molecules within the reaction.

In some embodiments, a system is provided that includes one or more data processors and a non-transitory computer readable storage medium containing instructions which, when executed on the one or more data processors, cause the one or more data processors to perform part or all of one or more methods disclosed herein.

In some embodiments, a computer-program product is provided that is tangibly embodied in a non-transitory machine-readable storage medium and that includes instructions configured to cause one or more data processors to perform part or all of one or more methods disclosed herein.

The invention comprises a system as set forth in claim 1. In some embodiments, the system includes a non-transitory computer readable storage medium containing instructions which, when executed on the one or more data processors, cause the one or more data processors to perform part or all of one or more methods and/or part or all of one or more processes disclosed herein. The invention further comprises a computer-program product as set forth in claim 15.

BRIEF DESCRIPTION OF THE DRAWINGS

The present invention will be better understood in view of the following nonlimiting figures, in which:

FIG. 1 shows an interaction system for configuring and using a simulation to facilitate subsequent experiment configurations according to various embodiments;

FIG. 2 shows a representation of modules representing distinct biological functions according to various embodiments;

FIG. 3 shows a simulation controller that dynamically integrates results generated by different types of models to simulate higher-level states and reactions according to various embodiments;

FIG. 4 shows a process for dynamically synthesizing results generated by multiple simulators to simulate higher-level results according to various embodiments;

FIG. 5 shows a module-specific simulation controller to simulate states and reactions according to various embodiments;

FIG. 6 shows a process for using a simulator to generate metabolite time-course data according to various embodiments;

FIG. 7 shows a typical enzymatic reaction according to various embodiments;

FIG. 8 shows a binding-dissociation step of a reaction according to various embodiments;

FIG. 9 shows a transition step of a reaction according to various embodiments;

FIG. 10 shows a binding component and a transition component according to various embodiments;

FIG. 11 shows a typical enzymatic reaction decomposed into individual components according to various embodiments;

FIG. 12 shows a typical enzymatic mechanism decomposed into individual components according to various embodiments;

FIG. 13 shows a typical enzymatic mechanism as a single path through a free energy space according to various embodiments;

FIG. 14 shows a competitive inhibition mechanism decomposed into individual components according to various embodiments;

FIG. 15 shows potential catalog or knowledge base of enzymatic mechanisms with several levels of complexity decomposed into individual components according to various embodiments;

FIGS. 16A-16E show how an entire biological system, biochemical process, or biochemical pathway 1can be represented by multiple enzymatic mechanisms composed using various combinations of binding and transition components according to various embodiments;

FIG. 17 shows a first standardized mathematical construct that may be used to model a single binding component (binding or dissociation) and a second standardized mathematical construct that may be used to model a single transition component according to various embodiments;

FIGS. 18A-18D show a process flow for configuring components and sets of standardized mathematical constructs using an interaction system to simulate the overall behavior of a system according to various embodiments;

FIG. 19 shows the free energy states for a typical enzyme reaction according to various embodiments;

FIG. 20 shows the free energy $\Delta G$ for a binding step of a reaction according to various embodiments;

FIG. 21 shows the free energy $\Delta G$ for a transition step of a reaction according to various embodiments;

FIG. 22 shows the reaction rate V determined for each simulation being plotted as a function of substrate concentration [S] according to various embodiments;

FIG. 23 shows a best-fit line curve can be generated to connect the data points of each simulation and graph the reaction rate V as a function of substrate concentration [S] according to various embodiments;

FIG. 24 shows a process flow for configuring components and sets of standardized mathematical constructs using an interaction system to simulate the overall behavior of a system according to various embodiments;

FIG. 25 shows how waypoints within a free energy $\Delta G$ profile may be learned by a prediction model according to various embodiments;

FIG. 26A shows waypoints of a free energy $\Delta G$ profile and in silico behavior of a reaction compared to expected behavior of the reaction prior to fitting the curves and learning the waypoints according to various embodiments;

FIG. 26B shows waypoints of a free energy $\Delta G$ profile and in silico behavior of a reaction compared to expected behavior of the reaction after fitting the curves and learning the waypoints according to various embodiments;

FIG. 27 shows a process flow for modeling a reaction pathway, process, or system according to various embodiments;

FIG. 28 shows a process flow for predicting parameters and parameterizing a model for a reaction according to various embodiments; and

FIG. 29 shows an example computing device suitable for modeling in silico the kinetics of systems of connected biochemical reactions according to various embodiments.

In the appended figures, similar components and/or features can have the same reference label. Further, various components of the same type can be distinguished by following the reference label by a dash and a second label that distinguishes among the similar components. If only the first reference label is used in the specification, the description is applicable to any one of the similar components having the same first reference label irrespective of the second reference label.

DETAILED DESCRIPTION

Specific details are given in the following description to provide a thorough understanding of the embodiments. However, it will be understood that the embodiments may be practiced without these specific details. For example, circuits, systems, networks, processes, and other components may be shown as components in block diagram form in order not to obscure the embodiments in unnecessary detail. In other instances, well-known circuits, processes, algorithms, structures, and techniques may be shown without unnecessary detail in order to avoid obscuring the embodiments.

Also, it is noted that individual embodiments may be described as a process which is depicted as a flowchart, a flow diagram, a data flow diagram, a structure diagram, or a block diagram. Although a flowchart or diagram may describe the operations as a sequential process, many of the operations may be performed in parallel or concurrently. In addition, the order of the operations may be re-arranged. A process is terminated when its operations are completed, but could have additional steps not included in a figure. A process may correspond to a method, a function, a procedure, a subroutine, a subprogram, etc. When a process corresponds to a function, its termination may correspond to a return of the function to the calling function or the main function.

## I. Introduction

The following disclosure describes a general and scalable techniques for modeling in silico the kinetics of systems of connected biochemical reactions. General meaning that core "building block" components can be assembled to represent the detailed mechanism of most biochemical processes such as enzymatic reactions. Scalable meaning that these components can be systematically translated into standard mathematical constructs regardless of the size of the biological system.

A fundamental challenge to the modeling of a biological system such as a whole cell is integrating the kinematic behavior of individual enzymes and enzymatic reactions to the cellular level over several spatial and temporal scales (i.e., predict the rate at which each reaction in a system is running in parallel given any condition). This is challenging because it requires accurate parameter values and scalable methods for simulating large models. To overcome this fundamental challenge, biochemical systems have been modeled using a simplified irreversible reaction shown in Equation (1) known as the Michaelis-Menten equation, which only requires three kinetic parameters.

$$E + S \underset{k_{-1}}{\overset{k_1}{\rightleftharpoons}} ES \overset{k_2}{\dashrightarrow} E + P$$

$$\text{Equation (1)}$$

where S, E, ES, and P denote the substrate, enzyme, enzyme-substrate complex, and product, respectively. The full mass action description of this reaction requires the three kinetic parameters: $k_1$, $k_{-1}$ and $k_2$, which are the forward association, dissociation and catalytic rate parameters, respectively.

Although the Michaelis-Menten equation provides an approximation of the single enzyme action, in vitro studies of single molecule enzyme kinetics have shown that this approximation is only sufficient in experiments where there is only one substrate/product and no initial product at the start of the system. However, there is a desire to model systems with in vivo conditions that provide a product at the start and/or multiple substrate/products. To overcome these limitations, modifications to the Michaelis-Menten equation have led to other enzyme kinetic equations and models such as a reversible Michaelis-Menten model and the Tzafriri total quasi-steady state assumption (tQSSA) model. Whilst the reversible Michaelis-Menten model is more widely used, it is strictly accurate at low enzyme concentrations. Since this may not be true under many in vivo conditions, unrealistic conclusions may be drawn from biological system models using the reversible Michaelis-Menten model. The tQSSA model is not subject to the same limitation as the reversible Michaelis-Menten model, but the tQSSA model has a more complex mathematical form that requires reanalysis for each distinct network to which it is applied. Currently, system and process modelers must choose between complex enzyme kinetics models with high parameter dimensionality, or simpler enzyme kinetics models at the cost of accuracy.

To address these limitations and problems, the biochemical reaction modeling techniques disclosed herein provide a balance between complex enzyme kinetics models and simpler enzyme kinetics models by breaking an enzymatic reaction mechanism into component steps, where the algorithm or mathematics for each component step are expressed in a simple, uniform and scalable manner. The kinetic parameters used for each component step are tied to fundamental thermodynamics using thermodynamic profiles that can be learned by fitting in silico enzymatic behavior to known terms utilized in the description of an enzyme or enzymatic reaction (e.g., Vmax, which is a constant that describes the turnover rate of an enzyme-substrate complex to product and enzyme, and Km which is the Michaelis constant that describes the amount of substrate needed for the enzyme to obtain half of its maximum rate of reaction). Thereafter, the kinetics of the enzymatic reaction mechanism can be modeled and simulated based on the core components and learned parameters. By iterating this process (e.g., by repeating the process of breaking each enzymatic reaction mechanism into its core components, learning the kinetic parameters using fundamental thermodynamics, and modeling the kinetics of the enzymatic reaction mechanism based on the core components and learned parameters), an accurate model and simulation of a biological system or biochemical process may be generated.

One illustrative embodiment of the present disclosure is directed to a system that includes one or more data processors and a non-transitory computer readable storage medium containing instructions which, when executed on the one or more data processors, cause the one or more data processors to perform actions including: deconstructing a reaction into a plurality of component steps, where the reaction is part of a pathway or process in a system to be modeled; translating each component step of the plurality of component steps into a set of rate equations to obtain a standard mathematical construct (i.e., a mathematical model) representing each component step, where the set of rate equations comprise a forward rate equation and a reverse rate equation, and where each forward rate equation and each reverse rate equation is a first-order rate equation or a second-order rate equation; numerically integrating across the standard mathematical constructs using a system of ordinary differential equations to determine a contribution of each component step of the plurality of component steps to a rate of change of molecules within the pathway or process; and deriving a in silico behavior of the system based on the contribution of each component step of the plurality of component steps to the rate of change of the molecules within the pathway or process.

Another illustrative embodiment of the present disclosure is directed to a system that includes one or more data processors and a non-transitory computer readable storage medium containing instructions which, when executed on the one or more data processors, cause the one or more data processors to perform actions including: obtaining an initial energy profile for a reaction, where the reaction is part of a pathway or process in a system to be modeled; deriving forward and reverse rate constants for each component step of a plurality of component steps representing the reaction based on the initial energy profile, where each component step is represented by a standard mathematical construct comprising a set of forward and reverse rate equations that apply the forward and reverse rate constants to one or more concentrations of molecules, respectively; deriving a in silico behavior of the reaction based on the contribution of each component step of the plurality of component steps to a rate of change of the molecules within the reaction, where the contribution of each component is determined from the standard mathematical construct applying the forward and reverse rate constants to the one or more concentrations of molecules, respectively; predicting, using a prediction model, a new energy profile for the reaction based on the derived in silico behavior of the reaction, where the predicting comprises learning a set of parameters including waypoints or free energy states, which are changeable to define a path of the reaction from substrate to product to fit the in silico behavior of the reaction to an expected behavior of the reaction; deriving updated forward and reverse rate constants for each component step of the plurality of component steps representing the reaction based on the new energy profile; and deriving an updated in silico behavior of the reaction based on the contribution of each component step of the plurality of component steps to the rate of change of the molecules within the reaction, where the contribution of each component is determined from the standard mathematical construct applying the updated forward and reverse rate constants to the one or more concentrations of molecules, respectively.

As used herein, the terms "substantially," "approximately" and "about" are defined as being largely but not necessarily wholly what is specified (and include wholly what is specified) as understood by one of ordinary skill in the art. In any disclosed embodiment, the term "substantially," "approximately," or "about" may be substituted with "within [a percentage] of" what is specified, where the percentage includes 0.1, 1, 5, and 10 percent. As used herein, when an action is "based on" something, this means the action is based at least in part on at least a part of the something.

Advantageously, these approaches provide a workflow for modeling the kinematic behavior of a system of reactions. The approaches are general enough for any biochemical system, and scale easily in terms of size and/or complexity. The approaches take advantage of available data and simple mathematic constructs and don't become so involved in missing data and difficult or complicated mathematics that it becomes nearly impossible to model the kinematic behavior of a system of reactions.

## II. <ins>Interaction System</ins> and <ins>Biological System</ins> <ins>Modeling Techniques</ins>

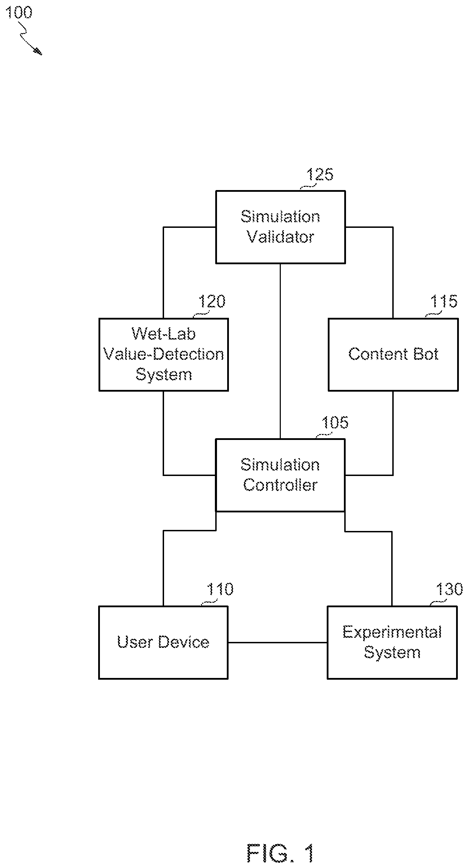

FIG. 1 shows an interaction system 100 for configuring instances or versions of a model and using a simulation to facilitate subsequent experiment configurations (e.g., simulation of a biological system's response to a new demand) according to various embodiments. Each instance of the models may have a combination of modules, perturbations (such as knockouts), and may be built using a particular set of experimental data. In order to facilitate the configuring of a model (e.g., a biological system) and simulate an outcome of the model, the interaction system 100 can include one or more components, each of which can include (for example) one or more servers, one or more computers and/or one or more mobile devices. In some instances, two or more of the components can be included in a same server, same server system, same computer, etc. Interaction system 100 can include one or more networks (e.g., a wired network, a wireless network, the Internet, a local area network, a wide area network, a short-range network, etc.), such that each component in the interaction system 100 can communicate with one or more other components in the interaction system 100.

Interaction system 100 can include a simulation controller 105 that defines, generates, updates and/or executes each of one or more simulations. A simulation can be configured to simulate dynamic progression through states, a time-evolved state of a model of a biological system and/or a steady state based on an iterative module-based assessment. It will be appreciated that identifying a steady-state and/or balanced solution for a module at a given time step need not indicate that a steady-state and/or balanced solution has been, can be or will be identified for the model in general (e.g., as metabolites produced and/or consumed at one module may further be produced and/or consumed at another module that need not be configured for balancing fluxes).

A given model can be used to generate and run any number of simulations. Differing initial conditions and/or differing automatically generated values in stochastic portions of the simulation (e.g., generated using a pseudo-random number generation technique, a stochastic pull from a distribution, etc.) can result in different output results of different simulations. The biological system model can be made up of one or more modules, and during a simulation run, each module is run independently and passes results back up to the biological system model level. More specifically, the biological system (e.g., a whole cell) may be modeled in accordance with a coordinated operation of multiple modules that represent structure(s) and/or function(s) of the biological system. Each module may be defined to execute independently, except that a shared set of state values (e.g., a state vector) maintained at the biological system model level may be used and accessed by multiple modules at each time point.

In some instances, each module of the biological system is configured to advance across iterations (e.g., time points) using one or more physiological and/or physics-based models (e.g., flux balance analysis (FBA), template synthesis, bulk-mass flow analysis, constant non-specific degradation, empirical analysis, etc.). The module-specific iteration processing can further be based on one or more module-specific state values (as determined based on an initial definition for an initial iteration processing or a result of a previous iteration processing for a subsequent iteration processing). The module-specific iteration processing can further be based on one or more parameters defined for the module that are fixed and/or static across iterations across iterations.

Simulation controller 105 can generate simulation configurations using one or more inputs received from a user device 110. For example, simulation controller 105 may generate an interface (or may at least partly define specifications for an interface) that is to be availed and/or transmitted to user device 110 and to include input fields configured to receive inputs that correspond to a selection of (for example) one or more modules to be used for a given biological system model, a model type to be used for each of the one or more modules, one or more parameters that are to be effected by a given module's model and used during execution, and/or one or more initial state-value definitions that are to be used by a given module's model and used during execution. In some instances, the interface identifies a default value for each of one, more or all parameters of the model and for each of one, more or all of the initial-state values of the model and is configured to receive a modification to a subset or all of the parameters and/or initial-state values for which a default value was identified. In some instances, modifying a default initial-state value and/or parameter can correspond to a perturbation of performance of a corresponding module and/or the biological system.

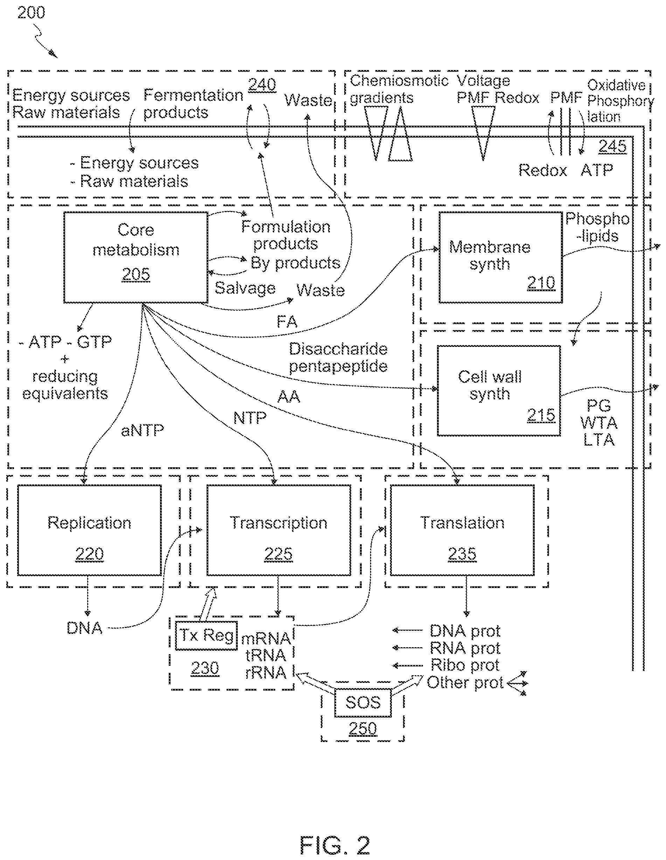

As another example, the interface may further or alternatively be configured to receive an input that corresponds to a selection of one or more default modules and a selection of a model type to be used for each of one or more modules. For example, the interface may include one or more modules (as shown in FIG. 2) representing distinct biological functions in a biological system model, and for each module: a name of the module, a default model type for the module and an option configured to receive a selection of another model type for the module (e.g., that identifies one or more other model types that can be selected for the module).

Default structure of a simulation (e.g., corresponding to default modules, default parameters, default initial-state values and/or default model selections) can be determined based on detected internal or external content and/or based on lab results (e.g., results from physical experiments). The content can include (for example) online, remote and/or local content that is collected by a content bot 115. Content bot 115 can (for example) include a crawler that performs a focused crawling and/or focused browsing (for example) the Internet, a part of the Internet, one or more pre-identified websites, a remote (e.g., cloud-based) storage system, a part of a remote storage system, a local storage system and/or a part of a local storage system. The crawling can be performed in accordance with one or more crawling policies and/or one or more queries that corresponds to one or more modules and/or models (e.g., where each query includes a variable name, representation or description and/or a cellular-function name, .representation or description).

The lab results can be received from a wet-lab value detection system 120, which can be configured to trigger performance of one or more investigations (e.g., physical experiments) to detect and/or measure data corresponding to an initial-state value and/or data corresponding to a characteristic or parameter of a biological system. Wet-lab value-detection system 120 can transmit one or more results of the investigation(s) back to simulation controller 105, which may thereafter determine and/or define a default initial-state value or parameter or a possible modification thereof based on the result(s).

Interaction system 100 further includes a simulation validator 125, which can be configured to validate performance of a simulation. The validation may be performed based on pre-identified indications as to how a biological system functions normally and/or given one or more perturbations. Such indications can be defined based on content collected from content bot 115 and/or results from wet-lab value-detection system 120. The data used to validate the simulation may include (for example) one or more balanced values, one or more values indicative of cell dynamics, one or more steady-state values, one or more intermediate values and/or one or more time-course statistics. Simulation validator 125 may return a performance result that includes (for example) a number, category, cluster or binary indicator to simulation controller 105. Simulation controller 105 may use the result to determine (for example) whether a given simulation configuration is suitable for use (e.g., in which case it may be selectable in an interface).

After a simulation is configured with definitions and/or selections of modules, module-specific models, parameters and/or initial-state values, simulation controller 105 can execute the simulation (e.g., in response to receiving an instruction from user device 110 to execute the simulation). The simulation execution can produce one or more simulation results, which may include (for example) one or more balanced values, kinetic values, etc. For example, the simulation can identify a solution for a set of reaction-corresponding stoichiometric equations using linear algebra, such that production and consumption of metabolites represented in the equations is balanced. Notably, this balance may be specific to a given module and need not be achieved for all metabolites produced or consumed by reactions for a given module (e.g., as a non-zero net production or consumption of one or more boundary metabolites may be pre-defined and/or a target result for a module). Simulation controller 105 can transmit the results (e.g., via an interface) to user device 110.

In some instances, the results can be used to trigger and/or define a subsequent experiment. For example, simulation controller 105 may determine whether a given predefined condition is satisfied based on the results and, if so, may transmit simulation-specific data (e.g., indicating one or more initial-state values, parameters, mutations corresponding to simulation definitions, etc.) to an experimental system 130. The transmission may be indicative of and/or include an instruction to perform an experiment that corresponds to the simulation.

As another example, upon receiving simulation results from simulation controller 105, user device 110 can present an interface that includes some or all of the results and an input component configured to receive input corresponding to an instruction to perform an experiment that corresponds to the simulation. Upon receiving a selection at the input component, user device 110 may transmit data corresponding to the simulation to experimental system 130. After performing a requested experiment, experimental system 130 may return one or more results to simulation controller 105 and/or user device 110.

FIG. 2 shows an illustrative representation of given biological system model 200. The overall modeling strategy includes partitioning the biological system model 200 into modules that can be modeled separately, using a methodology and level of detail appropriate to and/or selected for each module. The partitioning and level of detail for each module can be selected based on (for example) the experiments or simulations that are to be run by the model (e.g., the questions trying to be solved by the model). The selection may be made by the modeler and/or computing system (e.g., the interaction system 100 described with respect to FIG. 1). For example, a user working through an interface of an integrated development environment, a script, and/or an automated system may be implemented to select one or more modules and select a model type to be used for each of one or more modules to ultimately generate the biological system model 200. Additionally or alternatively, the partitioning can be customized and depend on an assessment of the biological functions defined for the initial high-level data set. For example, a separate module may be defined to represent each of the following biological functions: core metabolism 205, membrane synthesis 210, cell-wall synthesis 215, DNA replication 220, transcription 225, transcription regulation 230, translation 235, RNA salvage (not shown), protein and RNA maturation, protein salvage (not shown), transmembrane transport 240 (including electron chain, oxidative phosphorylation, redox, and pH interconversion activity 245), signal transduction (not shown), stress response and growth rate regulation 250, cell division, chemotaxis (not shown), and cell-cell signaling (not shown).

Biological system model 200 can include at least one module that handles core metabolism 205. One possible core metabolic module uses an FBA model, which takes its general shape from standalone FBA, but includes modifications that account for interactions of the core metabolic module with other modules. Each of one, more or all other modules may have their own production and consumption of some of the same molecules within the FBA network, as described in further detail herein. However, as should be understood to those of ordinary skill in the art, an FBA model does not have to be incorporated into the overall biological system model 200 in order for every simulation to work. Instead, various types of models can be used for the modules (e.g., core metabolism 205, membrane synthesis 210, cell-wall synthesis 215, etc.) so long as the type of models can be configured to read values from the state vector and return a list of changes that should be made to the state vector.

For one exemplary instantiation of biological system model 200, core metabolism 205, membrane synthesis 210, and cell-wall synthesis 215 may be encompassed as a single FBA problem, whereas DNA replication 220, transcription 225, transcription regulation 230, and translation 235 may be isolated from the rest of the metabolic network. Meanwhile, transcription 225 and translation 235 may use a template synthesis model, and DNA replication 220 may use a bulk mass-flow model. Transcription regulation 230 may be empirical and static. Optionally, RNA salvage may be modeled using constant non-specific degradation, polymerized DNA, RNA, and protein levels may be determined by the intrinsic rates of the processes that produce them, and the remainder of the components are provided as inputs or parameters of the model.

For another exemplary instantiation of biological system model 200, core metabolism 205 may be encompassed as a single FBA problem. The balance of internal metabolite pools and the supply of building blocks for other processes may be maintained by core metabolism 205. DNA replication 220, transcription 225, transcription regulation 230, and translation 235 may then be isolated from the rest of the metabolic network. Membrane biosynthesis 210 and cell-wall synthesis 215 may be modeled by substrate- and catalyst-driven kinetics. Import and export rates and all exchange with the environment may be driven by the kinetics of membrane transport. Transcription 225 and translation 235 may use a template synthesis model, and DNA replication 220 may use a bulk mass-flow model. Transcription regulation 230 may be empirical and static. Optionally, RNA salvage may be modeled using representations of constant non-specific degradation, while polymerized DNA, RNA, and protein levels may be determined by the intrinsic rates of the processes that produce them, and the remainder of the components for the biological system can be provided as inputs or parameters of the model.

For another exemplary instantiation of biological system model 200, core metabolism 205 may be encompassed as an FBA problem, whereas one or more of membrane synthesis 210, cell-wall synthesis 215, DNA replication 220, transcription 225, transcription regulation 230, and translation 235 can be isolated from the rest of the metabolic network. The balance of internal metabolite pools and the supply of building blocks for other processes may be maintained by core metabolism 205. Membrane biosynthesis 210 and cell-wall synthesis 215 may be modeled by substrate and catalyst driven kinetics. Import and export rates, and all exchange with the environment may be driven by the kinetics of membrane transport. Redox balance, pH, and chemiosmotic gradients may be maintained explicitly. DNA replication 220, transcription 225 and translation 235 may use models based on initiation, elongation, and termination, Transcription regulation 230 may be pattern driven. Stress response and growth rate regulation 250 may be modeled using feedback control mechanisms. Optionally, RNA salvage may be modeled using constant non-specific degradation, while polymerized DNA, RNA, and protein levels may be determined by the intrinsic rates of the processes that produce them, and the remainder of the components for the biological system can be provided as inputs or parameters of the model.

While the biological system model 200 has been described at some length and with some particularity with respect to several described modules, combinations of modules, and simulation techniques, it is not intended that the biological system model 200 be limited to any such particular module configuration or particular embodiment. Instead, it should be understood that the described embodiments are provided as examples of modules, combinations of modules, and simulation techniques, and the modules, combinations of modules, and simulation techniques are to be construed with the broadest sense to include variations of modules, combinations of modules, and simulation techniques listed above, as well as other modules, combinations of modules, and simulation techniques configurations that could be constructed using a methodology and level of detail appropriate to each module and the biological system model 200.

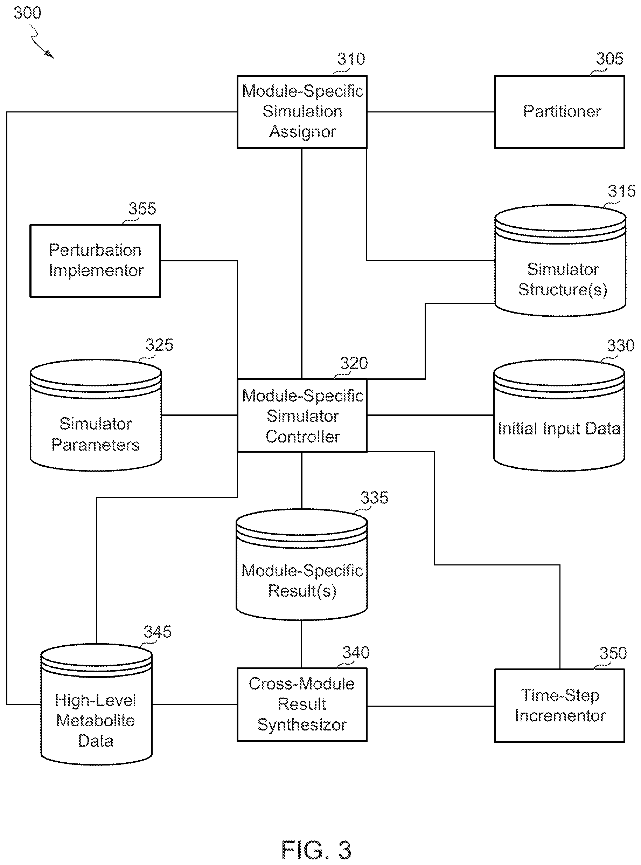

FIG. 3 shows a simulation controller 300 that dynamically integrates results generated by different types of models configured by an integrated development environment (e.g., the interaction system 100 described with respect to FIG. 1) to simulate higher-level states and reactions of a biological system model (e.g., biological system model 200 as described with respect to FIG. 2) according to various embodiments. A partitioner 305 that can identify one or more modules to potentially use for a simulation. In some instances, the modules are identified to correspond to distinct biological functions or physiological processes within a biological system model. Nonetheless, at least one module (e.g., a core module) may address in more detail or cover a larger set of biological functions (e.g., correspond to a core level of physiology across the biological system such as general metabolism of the biological system), whereas at least one other module (e.g., a non-core module) may address in less detail or cover a smaller set biological function (e.g., correspond to transcription and/or translation).

A module-specific simulation assignor 310 may assign, to each module, a simulation type. The simulation type can be selected from amongst one or more types that are associated with the module and/or corresponding physiological process. The one or more types may differ with regard to (for example) a degree of detail to which a physiological process is modeled and/or how the process is modeled. For example, the one or more types may include a simulation using a metabolism-integrated model (e.g., in which specific end products are added to an objective function of a metabolism-based model), substrate- and/or catalyst-drive model using kinetic parameters and reactions, and/or higher-order structure model. A structure for each simulation type (e.g., that indicates how the simulation is to be performed and/or program code) is included in a simulator structure data store 315. Simulator structure data store 315 can further store an association between each simulation type and one or more modules for which the simulation type is associated and is permitted for selection for use.

A module-specific simulator controller 320 can identify, for each module, one or more simulation parameters and an input data set. The simulation parameters may be retrieved from a local data store (e.g., a simulator parameters data store 325) or from a remote source. Each of one or more of the simulation parameters may have been identified based on (for example) user input, a data-fitting technique and/or remote content. The parameter(s), once selected, may be fixed across time-step iterations.

At an initial time step, the input data set can include one or more initial input values, which may be retrieved from a local data store (e.g., an initial input data store 330) or from a remote source. Each of one or more of the initial input values may have been identified based on (for example) user input, a data-fitting technique and/or remote content. With respect to each subsequent time step, the input data set can include (for example) one or more results from a previous iteration of the module and/or one or more high-level results (e.g., cumulative or integrated results) generated from a previous iteration of the multi-module simulation. For example, a module-specific results data store 335 may store each of one, more or all results generated by the assigned simulation for each of one, more or all past time steps, and at least one of the stored results associated with a preceding time step (e.g., most recent time step) can be retrieved.

Upon identifying the input data set and parameters, module-specific simulator controller 320 can run the simulation assigned to the module. Execution of module-specific simulations may be performed concurrently, in parallel and/or using different resources (e.g., different processors, different memory and/or different devices). Results of the simulation run can be stored in module-specific results data store 335.

After results have been generated for each module, a cross-module result synthesizor 340 can access the module-specific results (from one or more module-specific results data stores or direct data availing) and synthesize the results to update high-level data such as a state vector (e.g., stored in a high-level metabolite data store 345). For example, a set of results generated by different modules but relating to a same variable may be identified. The results may be integrated by (for example) summing variable changes as indicated across the results (e.g., potentially with the implementation of one or more caps pertaining to a summed change or to a value of a variable after the summed change is effected). In some instances, a hierarchy is used, such that a result from one module (if available or if another condition is met) is to be exclusively used and a result from another module is to otherwise be used.

Upon synthesizing the results, a time-step incrementor 350 can increment a time step to a next time step so long as the simulation has not completed. It may be determined that the simulation is complete when (for example) processing for a predefined number of time steps has been performed, a particular result is detected (e.g., indicating that a target cell growth has occurred or that a cell has died) or steady state has been reached (e.g., as indicated by values for one or more predefined types of results differing by less than a predefined threshold amount across time steps). When the time step is incremented, module-specific simulator controller 320 can, for each module, collect a new input data set and run the assigned simulation. When the simulation is complete, an output can be generated to include one or more module-specific results, some or all high-level data and/or processed versions thereof. For example, the output may include time-course data for each of one or more metabolites, growth of the biological system over a time period (e.g., as identified by a ratio of availability values of one or more particular metabolites at a final time step as compared to availability values at an initial time step) and/or a growth rate. The output can be transmitted to another device (e.g., to be presented using a browser or other application) and/or presented locally.

Multi-module simulation controller 300 can also include a perturbation implementor 355. Perturbation implementor 355 can facilitate presentation of an interface on a user device. The interface can identify various types of perturbations (e.g., mutations). Perturbation implementor 355 may facilitate the presentation by transmitting data (e.g., HTTP data) to a user device, such that the interface can be presented online. Perturbation implementor 355 can detect a selection that corresponds to a particular perturbation and can send an indication to module-specific simulator controller 320. Module-specific simulator controller 320 can use functional gene data to determine how the mutation affects one or more metabolites and/or one or more simulated processes. A structure of a simulator, one or more simulator parameters and/or one or more initial-input values may then be adjusted in accordance was the perturbation's effects. Thus, multi-module simulation controller 300 can generate output that is indicative of how the perturbation affects (for example) physiological processes and/or growth of the biological system.

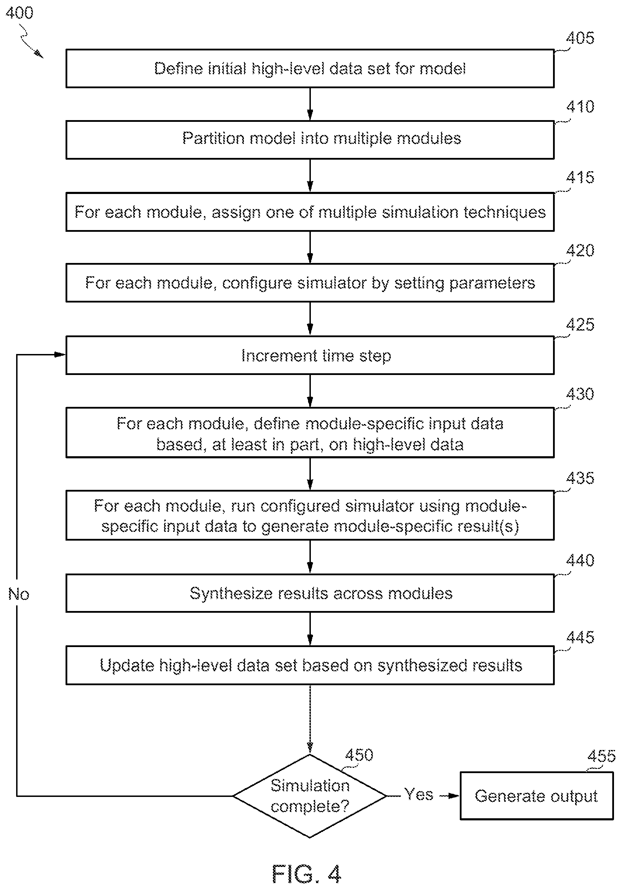

FIG. 4 shows a process 400 for dynamically synthesizing results generated by multiple simulators to simulate higher-level results according to various embodiments. In some embodiments, the processes depicted in process 400 are implemented by the interaction system 100 of FIG. 1, and discussed with respect to the simulation controller 300 of FIG. 4. Process 400 begins at block 405 at which an initial high-level data set is defined for a biological system model. The initial high-level data set can identify (for example) variables, which may be referred to as the state of the biological system model or the state of the simulation, and these variables may be structured as a data structure (e.g., a state vector) and updated throughout a simulation run. In some instances, the variables include an initial availability of each of a set of molecules such as metabolites. The initial availability may be defined based on (for example) a default value, user input, data extracted from content (e.g., online content, remote content or local content that pertains to the molecules), etc. In some instances, the initial availability is determined based on whether any perturbation was identified (e.g., via user input) for a given simulation. If a perturbation was identified, the initial availability may be determined based on a particular perturbation that was identified and by using (for example) a look-up table to determine for which molecule(s) the perturbation affects an availability value and characteristics of such effect.

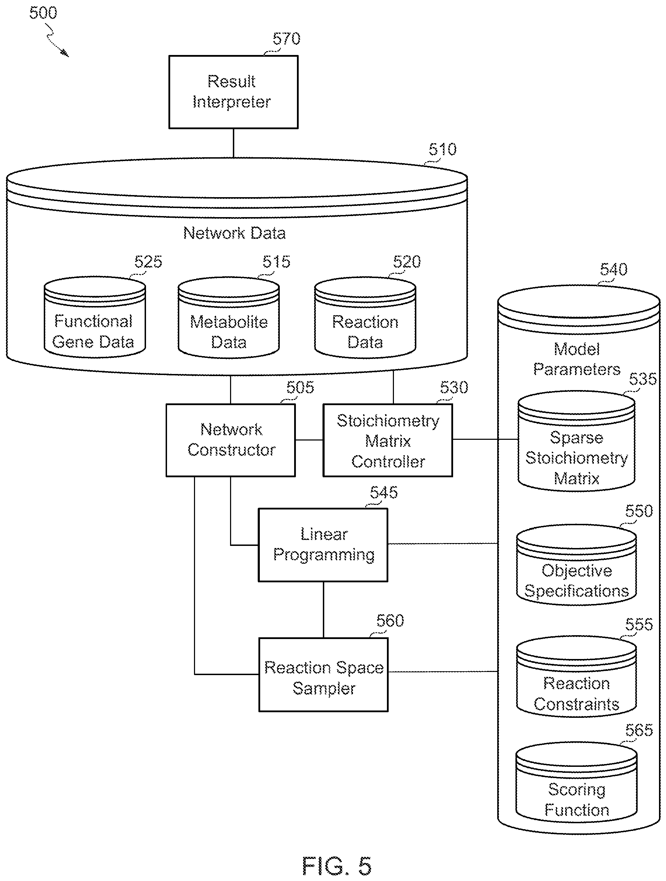

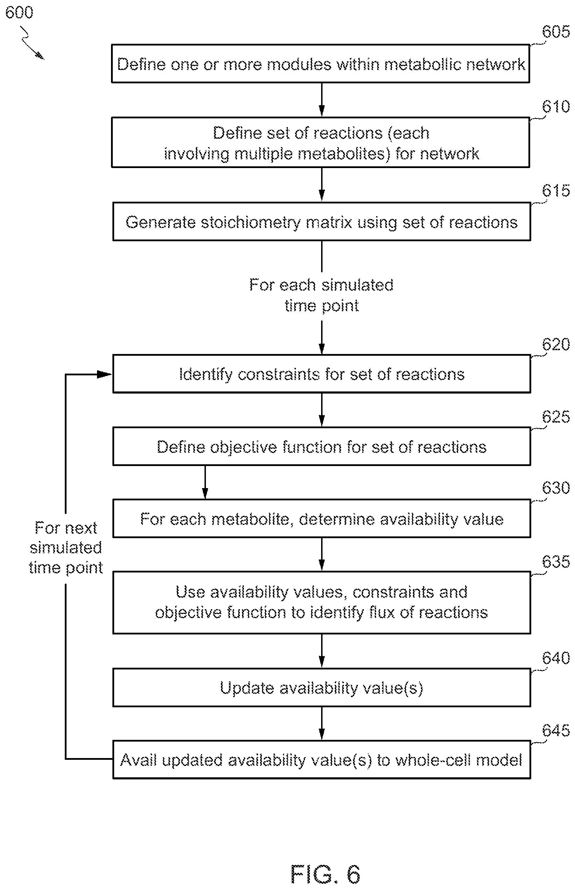

At block 410, a biological system model (e.g., a whole cell model) is partitioned into multiple modules. The partitioning can depend on metabolite dependencies and/or biological-functioning assessment. For example, a separate module may be defined to represent each of the following biological functions: core metabolism, membrane synthesis, cell-wall synthesis, DNA replication, transcription, transcription regulation, translation, RNA salvage, protein and RNA maturation, protein salvage, transmembrane transport (including electron chain, oxidative phosphorylation, redox, and pH interconversion activity), signal transduction, stress response and growth rate regulation (SOS), cell division, chemotaxis, and cell-cell signaling, as discussed in further detail with respect to FIG. 2. In some instances, two or more of these functions may be represented in a core module that models cell composition and growth using a single model. Particular cellular functioning need not be explicitly modeled and instead dynamics of end products of the particular cellular functioning may be modeled. For example, a core module may use a flux-based analysis or a simulation technique as described herein (e.g., in relation to FIG. 5 or FIG. 6).

In some instances, the partitioning may be performed based on user input and/or one or more default configurations. For example, an interface may be presented that identifies each potential separate module (e.g., an interface may be presented via simulation controller 105 as described with respect to FIG. 1). A default configuration may be to integrate the module into a core module (e.g., a core metabolism module) unless a contrary input is received or to perform a simulation using modeling specific to the module unless a contrary input is received. For example, an interface may be configured to receive one or more selections of modules that are to be excluded from a core module and to then integrate each other module into the core module.

At block 415, for each module, one or more simulation techniques are assigned to the module. A simulation technique may include a model type. In some instances, a simulation technique that is assigned to a core module includes a flux-based analysis or other simulation technique, as described herein. In some instances, a simulation technique includes a mechanistic model, a kinetic model, a partial kinetic model, a substrate- and/or catalyst-driven model, and/or a structural model. The simulation technique may be assigned based on (for example) user input and/or one or more predefined default selections. For example, for each non-core module, a default selection may be predefined that represents particular functioning of the module, and for each core module, a default selection may be predefined that simulates dynamics of metabolites across a simulated time period. An interface may identify, for each module, the default selection along with one or more other simulation techniques that are associated with the module (e.g., with the association(s) being based on stored data and/or a predefined configuration). User input may then indicate that an alternative simulation technique is to be used for one or more modules.

At block 420, for each module, a simulator is configured by setting parameters and variables. The parameters (e.g., numeric values) may correspond to inputs to be used in the simulation technique assigned to the module and that are not changed across time steps of the simulation. The particular parameters may be determined based on (for example) stored data, content, a communication from another system and/or user input. The one or more module-specific or cross-module variables (e.g., identifying an initial availability of one or more metabolites) may correspond to inputs to be used in the simulation technique assigned to the module and may be changed across time steps of the simulation. For example, a parameter may be determined for a simulator that sets a minimum viable pH in the cytoplasm (below which the cell dies), and a variable may be identified that describes a current pH in the cytoplasm. The variable (current pH) might change throughout the simulation; however, the parameter (the minimum possible pH) would not change and remains fixed. An initial value of the pH variable may be identified, e.g., the value at the start of the simulation may be set in step 405 or if it is module specific then it may be set in step 420, and like the minimum pH parameter this would be used as an input into the simulation. The values of variables and parameters are both inputs, but the distinction is that variables can change from their initial values, and parameters are fixed throughout the simulation run.

At block 425, a time step is incremented, which can initially begin a given simulation. At block 430, for each module, module-specific input data is defined at least in part on the high-level data. More specifically, a high-level data structure may identify, for each of a set of molecules (e.g., metabolites), an availability value. Each availability value may initially be set to an initial availability value, which may thereafter be updated based on processing results from each module that relates to the molecule. For a given module, at each time step, a current availability value can be retrieved from the data structure for each molecule that pertains to the simulation technique assigned to the module. The module-specific input data may further include one or more lower-level values that are independent from processing of any other module. For example, one or more variables may only pertain to processing of a given module, such that the module-specific input data may further include an initial value or past output value that particularly and exclusively relates to the module.

At block 435, for each module, the configured simulator assigned to the module is run using the module-specific input data to generate one or more module-specific results. The one or more module-specific results may include (for example) one or more updated molecule availability values and/or a change in one or more availability values relative to corresponding values in the input data.

At block 440, results can be synthesized across modules. The synthesis may include summing differences across modules. For example, if a first module's results indicate that an availability of a given molecule is to be increased by 5 units and a second module's results indicate that an availability of the given metabolite is to be decreased by 3 units, a net change may be calculated as being an increase in 2 units. The net change can then be added to a corresponding availability value for the molecule that was used for the processing associated with the current time step and returned as a list of changes that should be made to the state vector. One or more limits may be applied to a change (e.g., to disallow changes across time steps that exceed a predefined threshold) and/or to a value (e.g., to disallow negative availability values and instead set the value to zero).

At block 445, the high-level data set is updated based on the synthesized results. The update can include adding data to a data structure such as a state vector from which one or more modules retrieve high-level data. The added data can include the synthesized results in association with an identifier of a current time step. Thus, the data structure can retain data indicating how an availability of a metabolite changed over time steps. It will be appreciated that alternatively the update can include replacing current high-level data with the synthesized data.

At block 450, it is determined whether the simulation is complete. The determination may be based on a number of time steps assessed, a degree to which data (e.g., high-level data) is changing across time steps, a determination as to whether a steady state has been reached, whether one or more simulated biological events (e.g., cell division or cell death) have been detected, etc. If the simulation is not complete, process 400 returns to block 425.

If the simulation is complete, process 400 continues to block 455, at which an output is generated. The output may include some or all of the high-level data and/or some or all of the module-specific results. For example, the output may include final availability values that correspond to a set of metabolites and/or a time course that indicates a change in the availability of each of one or more metabolites over the simulated time period. The output may be presented at a local device and/or transmitted to another device (e.g., for presentation).

FIG. 5 shows a module-specific simulation controller 500 to simulate states and reactions of modules configured by an integrated development environment (e.g., the interaction system 100 described with respect to FIG. 1) according to various embodiments. A network constructor 505 can be configured to use a model to simulate actions performed by a module of a biological system model (e.g., biological system model 200 as described with respect to FIG. 2). In some instances, the model is flux balance analysis, and/or the model is configured to solve for updated state values based on a set of equations that represent concentration changes in the network (e.g., a metabolic network). As should be understood to those of ordinary skill in the art, a biological system model such as a whole cell model does not have to include an FBA module. For example, from the framework described herein, biological processes such as core metabolism may be modeled that is completely different from FBA. In such an instance, part or all of the description and drawings pertaining to FIGS. 5 and 6 that is specific to FBA (e.g., objective functions, constraints, and linear programming) may not be relevant to that particular instantiation of the model or to simulations run with that model. However, many of the components and techniques described with respect to FIGS. 5 and 6 could be applied to simulate states and reactions of modules implemented by other models. For example, any module can read values from the state vector and return an indication of one or more changes that should be made to the state vector. The FBA module (if it's even present in a particular instantiation of the model) may read and return more values than any other model, but a module modeled with FBA need not be handled by the simulation controller 300 any differently from other modules and/or models described herein.

Network constructor 505 can access a set of network data (e.g., parameters and variables) stored in a network data store 510 to define the model. Metabolite data 515 can identify each metabolite of a metabolome. As used herein, a "metabolite" is any substance that is a product of metabolic action or that is involved in a metabolic process including (for example) each compound input into a metabolic reaction, each compound produced by a metabolic reaction, each enzyme associated with a metabolic reaction, and each cofactor associated with a metabolic reaction. The metabolite data 515 may include for each metabolite (for example) one or more of the following: the name of the metabolite, a description, neutral formula, charged formula, charge, spatial compartment of the biological system and/or module of the model, and identifier such as PubChem ID. Further, metabolite data 515 can identify an initial state value (e.g., an initial concentration and/or number of discrete instances) for each metabolite.

Reaction data 520 can identify each reaction (e.g., each metabolic reaction) associated with the model. For example, a reaction can indicate that one or more first metabolites is transformed into one or more second metabolites. The reaction need not identify one-to-one relationships. For example, multiple metabolites may be defined as reaction inputs and/or multiple metabolites may be defined as reaction outputs. The reaction data 520 may include for each reaction (for example) one or more of the following: the name of the reaction, a reaction description, the reaction formula, a gene-reaction association, genes, proteins, spatial compartment of the biological system and/or module of the model, and reaction direction. Further, the reaction data 520 can identify, for each metabolite of the reaction, a quantity of the metabolite, which may reflect the relative input-output quantities of the involved metabolites. For example, a reaction may indicate that two first metabolites and one second metabolite are input into a reaction and that two third metabolites are outputs of the reaction. The reaction data 520 can further identify an enzyme and/or cofactor that is required for the reaction to occur.

Functional gene data 525 can identify genes and relationships between genes, proteins, and reactions, which combined provide a biochemically, genetically, and genomically structured knowledge base or matrix. Functional gene data 525 may include (for example) one or more of the following: chromosome sequence data, the location, length, direction and essentiality of each gene, genomic sequence data, the organization and promoter of transcription units, expression and degradation rate of each RNA transcript, the specific folding and maturation pathway of RNA and protein species, the subunit composition of each macromolecular complex, and the binding sites and footprint of DNA-binding proteins. Network constructor 505 can use functional gene data and the availability of proteins encoded by those genes to update reaction constraints. One exemplary technique by which genomic data can be associated with reaction data is evaluating Gene-Protein-Reaction expressions (GPR), which associate reactions with specific genes that triggered the formation of one or more specific proteins. Typically a GPR takes the form (Gene A AND Gene B) to indicate that the products of genes A and B are protein sub-units that assemble to form a complete protein and therefore the absence of either would result in deletion of the reaction. On the other hand, if the GPR is (Gene A OR Gene B) it implies that the products of genes A and B are isozymes (i.e., each of two or more enzymes with identical function but different structure) and therefore absence of one may not result in deletion of the reaction. Therefore, it is possible to evaluate the effect of single or multiple gene deletions by evaluation of the GPR as a Boolean expression. If the GPR evaluates to false, the reaction is constrained to zero in the model.

A stoichiometry matrix controller 530 can use reaction data 520 to generate a stoichiometry matrix 535. Along a first dimension of the matrix, different compounds (e.g., different metabolites) are represented. Along a second dimension of the matrix, different reactions are represented. Thus, a given cell within the matrix relates to a particular compound and a particular reaction. A value of that cell is set to 0 if the compound is not involved in the reaction, a postive value if the compound is one produced by the reaction and a negative value if the compound is one consumed by the reaction. The value itself corresponds to a cofficient of the reaction indicating a quantity of the compound that is produced or consumed relative to other compound consumption or production involved in the reaction.

Because frequently relatively few reactions correspond to a given compound, stoichiometry matrix 535 can be a sparse stoichiometry matrix. Stoichiometry matrix 505 can be part of a set of model parameters (stored in a model-parameter data store 540) used to execute a module.

One or more modules may be configured to use linear programming 545 to identify a set of compound quantities that correspond to balancing fluxes identified in reactions represented in stoichiometry matrix 535. Specifically, an equation can be defined whereby the product of stoichiometry matrix 535 and a vector representing a quantity for each of some of the compound quantities is set to zero. (It will be appreciated that the reactions may further include quantities for one or more boundary metabolites, for which production and consumption need not be balanced.) There are frequently multiple solutions to this problem. Therefore, an objective function is defined, and a particular solution that corresponds to a maximum or minimum objective function is selected as the solution. The objective function can be defined as the product between a transposed vector of objective weights and a vector representing the quantity for each compound. Notably, the transposed vector may have a length that is equal to the first dimension of stoichiometry matrix 535, given that multiple reactions may relate to a same compound.

The objective weights may be determined based on objective specifications 550, which may (for example) identify one or more reaction-produced compounds that are to be maximized. For example, the objective weights can be of particular proportions of compounds that correspond to biomass, such that producing compounds having those proportions corresponds to supporting growth of the biological system.

Each reaction may (but need not) be associated with one or more of a set of reaction constraints 555. A reaction constraint may (for example) constrain a flux through the reaction and/or enforce limits on the quantity of one or more compounds consumed by the reaction and/or one or more compounds produced by the reaction.

In some instances, linear programming 545 uses stoichiometry matrix 535 and reaction constraints 550 to identify multiple solutions, each complying with the constraints. When multiple solutions are identified, objective specifications 550 can be used to select from amongst the potential solutions. However, in some instances, no solution is identified that complies with stoichiometry matrix 535 and reaction constraints 555 and/or the only solution that complies with the matrix and constraints is not to proceed with any reaction.

A solution can include one in which, for each of a set of metabolites, a consumption of the metabolite is equal to a production of the metabolite. That is not to say that this balance must be achieved for each metabolite, as a set of reactions involve one or more "boundary metabolites" for which this balance is not achieved. For example, glucose can be consumed at a given rate, and/or acetate can be produced at a given rate.

Reaction data 520 may further identify an objective function that identifies a target product (e.g., representing cell growth rate) that is to be maximized. The objective function can identify particular ratios of multiple reactant metabolites that must be available to produce the product. Strictly enforcing the objective function may result in simulating no growth if a single metabolite is not produced. An alternative approach is to define one or more objective functions configured such that production of each of multiple target reactant metabolites that relate to the target product is to be maximized. A higher level whole-cell model can evaluate the production of multiple target reactant metabolites to determine whether to and/or an extent to which to simulate growth. For example, depending on which target reactant metabolite(s) are not produced, the whole-cell model may nonetheless simulate cell growth, simulate cell growth at a reduced rate, simulate no growth, simulate unhealthy or impaired growth or simulate cell death.

For example, a reaction space can be defined based on stoichiometry matrix 535 and reaction constraints 555.

The space may have as many dimensions as there are reactions. Each dimension can be restricted to include only integer values that extend along a range constrained by any applicable constraint in reaction constraints 555. A reaction space sampler 560 can then determine, for each of some or all of the points within the reaction space, a cumulative quantity of each metabolite that would be produced based on the associated reactions. Reaction space sampler 560 can compare these quantities to those in the objective vector (e.g., by determining an extent to which proportions of compounds are consistent).

In these instances, a scoring function 565 can indicate how to score each comparison. For example if proportions of each of two potential solutions differ from the objective proportions by 2, but one potential solution differs by 2 for a single compound and another by 1 for each of two compounds, scoring function 565 can be configured to differentially score these instances. For example, different weights may be applied to different compounds, such that differences that affect a first compound are more heavily penalized than differences that affect a second compound. As another example, scoring function 565 may indicate whether a score is to be calculated by (for example) summing all compound-specific (e.g., weighted) differences, summing an absolute value of all compound-specific (e.g., weighted) differences, summing a square of all compound-specific (e.g., weighted) differences, etc. Reaction space sampler 560 can then identify a solution as corresponding to reaction coefficients that are associated with a highest score across the reaction space.

Network constructor 505 can receive results from each of linear programming 545 and/or reaction space sample 560. In some instances, linear programming 545 can further avail its results to reaction space sample 560. When a balanced solution is identified by linear programming 545, reaction space sampler 560 need not sample the reaction space and need not avail reaction-space results to network constructor 505.

Network constructor 505 can identify a solution as corresponding to one identified by linear programming 545 when a balanced solution is identified and as a highest-score potential solution identified by reaction space sampler 560 otherwise. The solution can then indicate the compounds produced by and consumed by the reactions performed in accordance with the solution-indicated flux. Network constructor 505 can update metabolite data 515 based on this production and consumption.

In some instances, a solution is identified for each of a set of time points rather than only identifying one final solution. The iterative time-based approach may be useful when module-specific simulation controller 500 is but one of a set of simulation controllers and metabolite data 515 is influenced by the performance of other modules. For example, metabolite data 515 may be shared across modules or may be defined to be a copy of at least part of a cross-module metabolite data set at each time point. The updates to the metabolites performed by network constructor 505 may then be one of multiple updates. For example, an update by network constructor 505 may indicate that a quantity of a specific metabolite is to increase by four, while a result from another module indicates that a quantity of the specific metabolite is to decrease by two. Then the metabolite may change by a net of +2 for the next time iteration.

A results interpreter 570 can generate one or more results based on the updated metabolite data 515. For example, a result may characterize a degree of growth between an initial state and a steady state or final time point. The degree of growth may be determined based on a ratio between values of one or more metabolites at a current or final time point relative to corresponding values at an initial (or previous) time point. The one or more metabolites may correspond to (for example) those identified in an objective function as corresponding to biomass growth. As another example, a result may characterize a time course of growth. For example, a result may identify a time required for metabolite changes that correspond to a representation of a double in growth or a time constant determined based on a fit to values of one or more time series of metabolite values. The result(s) may be output (e.g., locally presented or transmitted to a remote device, such as a user device). The output can facilitate a presentation of an interface that indicates one or more simulation characteristics (e.g., one or more default values in terms of initial-state values or reaction data and/or one or more effected perturbations).

Operation of module-specific simulation controller 500 can be influenced by particular simulated perturbations of the whole cell. For example, each perturbation may correspond to a particular type of genetic mutation. The perturbation may have been identified based on detecting user input (e.g., a selection and/or text input received via an interface) that defines the perturbation. One exemplary type of perturbation is a gene mutation. An effect of the perturbation may be determined based on functional gene data (e.g., to determine how an availability of one or more metabolites is affected). High-level metabolite data, simulator parameters and/or high-level constraints may then be accordingly set, constrained and/or defined based on the perturbation. This high-level perturbation can thus then influence operation of one or more lower level modules.

FIG. 6 shows a process 600 for using a simulator to generate metabolite time-course data according to various embodiments. In some embodiments, the processes depicted in process 600 are implemented by the interaction system 100 of FIG. 1, and discussed with respect to the module-specific simulation controller 500 of FIG. 5. Process 600 begins at block 605, at which a one or more modules within a metabolic network (e.g., of a biological system) are defined. The module(s) can be defined based on which parts of the network exhibit relative functional independence and/or correspond to substantial independence in terms of biological activity. In some instances, a default is to define each part of a cell as part of a core module unless a different module corresponding to particular types of actions and/or cell components is defined.

At block 610, a set of reactions is defined for the network. In some instances, the set of reactions are defined for the module (or each module) that corresponds to the default model type. The set of reactions can indicate how various molecules such as metabolites are consumed and produced through part of all of a life cycle of a biological system. Each reaction thus identifies one or more metabolites that are consumed, one or more metabolites that are produced and, for each consumed and produced metabolite, a coefficient (which may be set to equal one) indicating a relative amount that is consumed or produced. The reaction may further include an identification of one or more enzymes, one or my cofactors and/or one or more environmental characteristics that are required for the reaction to occur and/or that otherwise affects a probability of the reaction occurring or a property of the reaction. The reactions may be identified based on (for example) online or local digital content (e.g., from one or more scientific papers or databases) and/or results from one or more wet-lab experiments.

At block 615, a stoichiometry matrix is generated using the set of reactions. Each matrix cell within the matrix can correspond to a particular metabolite and a particular reaction. The value of the cell may reflect a coefficient of the particular metabolite within the particular reaction (as indicated in the reaction) and may be set to zero if it is not involved in the reaction. In some instances, metadata is further generated that indicates, for each of one or more reactions, any enzyme, co-factor and/or environmental condition required for the reaction to occur.

At block 620, one or more constraints are identified for the set of reactions. In some instances, identifying the constraints may include identifying values for one or more parameters. For example, for each of one or more or all of the set of reactions, a constraint may include a flux lower bound and/or a flux upper bound to limit a flux, a quantity of a consumed or produced metabolite, a kinetic constant, a rate of production or decay of a component such as RNA transcript, an enzyme concentration or activity, a compartment size, and/or a concentration of an external metabolite. The constraint(s) may be identified based on (for example) user input, online or local data, one or more communications from a wet-lab system, and/or learned from statistical inference.

At block 625, an objective function is defined for the set of reactions. The objective function may identify what is to be maximized and/or what is to be minimized while identifying a solution. The objective function may (for example) identify a metabolite that is produced by one or more reactions or a combination of metabolites that is produced by one or more reactions. The combination may identify proportions of the metabolites. However, the objective function can have a number of limitations and may fail to reflect supply and demand within the other modules. Thus, in some instances, a limited objective function can be constructed to include a set of target values for each molecule within the metabolic network. The target values can incorporate intrinsic-rate parameters, supply rates of molecules, the consumption rates of molecules, and the molecule concentrations into a measurement of target concentrations of the molecule given supply, demand, and an "on-hand" concentration of each molecule, which represents the concentration of a molecule immediately available to a reaction pathway. The target values may be calculated and incorporated into the objective function to produce the limited objective function. This may be in the form of calculating an absolute difference between the target value and the proportional flux contribution of each molecule. This may be in the form of scaling the proportional flux contribution of each molecule. This may be in the form of adding to the proportional flux contribution of each molecule. Any other mathematical modification of the proportional flux contribution of each molecule that adjusts this value by the target value may be used. The target values may be positive or negative. For purposes of unit conversion, so that target values can be included in the objective function and compared to the flux values, the target values may be constructed as rates.

At block 630, for each metabolite related to the set of reactions, an availability value is determined. For an initial value, the value may be identified based on (for example) user input, digital content and/or communication from another system. Subsequent values may be retrieved from a local or remote data object that maintains centralized availability values for the set of metabolites.

At block 635, the availability values, constraints and objective function are used to determine the flux of one, more or all of the set of reactions. The flux(es) may indicate a number of times that each of one, more or all of the reactions were performed in a simulation in accordance with the availability values, constraints and objective function. The flux(es) may be determined based on a flux-balance-analysis model. In some instances, the flux(es) may be determined based on a sampling of all or part of an input space representing different flux combinations and scoring each input-space using a scoring function.

At block 640, a centralized availability value of one or more metabolites is updated based on the determined flux(es). More specifically, for each metabolite, a cumulative change in the metabolite's availability may be identified based on the cumulative consumption and cumulative production of the metabolite across the flux-adjusted set of reactions. The centralized availability value of the metabolite can then be incremented and/or decremented accordingly.

In some instances, at least one the one or more modules defined at block 605 are to be associated with a model that does not depend on (for example) a stoichiometry matrix and/or flux based analysis and/or that is based on physiological modeling. One or more modules based on one or more different types of models can also, at each time point, identify a change in metabolite availability values, and such changes can also be used to update a local or remote data object with centralized availability values. With respect to each metabolite, updates in availability values may be summed to identify a total change and/or updated availability value. In some instances, limits are set with respect to a maximum change that may be effected across subsequent time steps and/or a maximum or minimum availability value for a metabolite.

At block 645, availability data is availed to a higher-level model. State vectors can then be updated based on data from multiple modules.

Some or all of blocks 620-645 may be repeated for each of multiple simulated time points in a simulation. Thus, at each time point, constraints can be updated based on state-vector information (e.g., representing availability of catalysts), an objective function can be defined (e.g., which may change across time points based on a configuration of a higher level objective), updated metabolite availability values can be determined, updated reaction fluxes can be identified, and further updated availability values can be determined. In some instances, a predefined number of simulated time points are to be evaluated and/or simulated time points corresponding to a predefined cumulative time-elapsing period are to be evaluated. In some instances, a subsequent simulated time point is to be evaluated until a predefined condition is satisfied. For example, a predefined condition may indicate that metabolite values for a current simulated time point are the same or substantially similar as compared to a preceding simulated time point or a preceding simulated time period.

With regard to a repeated iteration of block 630, it will be appreciated that an availability value determined for a given metabolite need not be equal to the corresponding updated availability value from the previous iteration of block 640 and/or the sum of the previously determined availability value adjusted by the identified flux pertaining to the metabolite. Rather, a processing of the previous time point with respect one or more other modules may have also resulted in a change in the metabolite availability, and/or a higher level constraint and/or processing may influence the availability. Thus, the availability value for a given metabolite determined at block 630 for a current time point may be equal to the availability value determined at block 630 for a preceding time point plus the cumulative updates to the availability value across modules, with any limits imposed.

While not shown in process 600, one or more variables can be output (e.g., transmitted to a user device). The variable(s) may include final values (e.g., availability values after all iterations have been performed), time-course values, high-level values and/or module-specific values. For example, the availability data may include, for each of one, more or all metabolites: an availability value (e.g., a final availability value) and/or a time course of the availability value. In some instances, the availability data is output with reference availability data. For example, when part or all of the processing performed to calculate the availability values was associated with a perturbation, the reference availability data may be associated with an unperturbed state. In some instances, a processed version of the availability data is output. For example, a comparison of availability values for particular metabolites across time points may be used to generate one or more growth metrics (e.g., a growth magnitude or rate), which may be output. Outputting the availability data can include (for example) locally presenting the availability data and/or transmitting the availability data to another device.

## III. **Decomposing Biochemical Reactions and Processes**

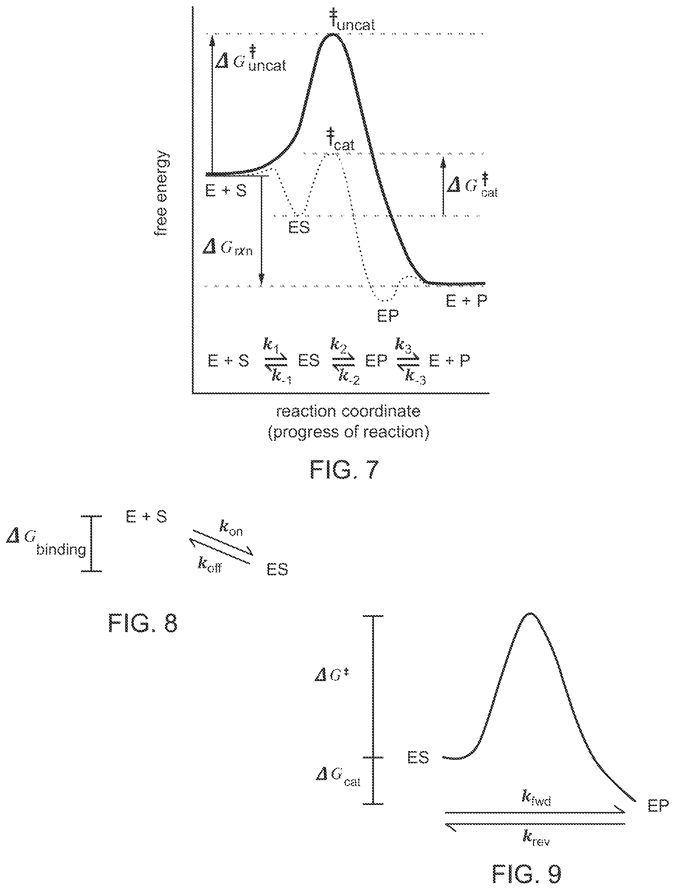

As shown in FIG. 7, the ability of enzymes (E) to catalyze reactions depends on their ability to interact directly and specifically with reactants. The reactant of an enzyme-catalyzed reaction is termed the substrate (S). An enzyme (E) works by binding to the substrate (S) to form an ES complex, then undergoing an internal state transition to an EP complex, which dissociates to release product (P) and regenerate free enzyme (E). The activation energy is lower for the ES $\Leftrightarrow$ EP transition than for uncatalyzed S $\Leftrightarrow$ P, which increases forward and reverse rates proportionally but does not affect overall $\Delta G$. In other words, the enzyme (E) provides the chemical context for the bound substrate (S) to have access to a low energy path to formation of products (P). The transition from substrate (S) to product (P) results in a net change in energy content $\Delta G$. The reaction passes through a transition state with activation energy $\Delta G\ddagger$. The $\Delta G\ddagger$ determines the rate of the reaction; where the higher the activation barrier, the slower the reaction. $\Delta G$ determines the ratio of the forward to reverse reaction rates, regardless of the activation barrier.

The mechanism of most enzymatic reactions can be broken down into three distinct steps including: (i) a binding-dissociation of the ES complex, (ii) a transition from the ES complex to the EP complex, and (iii) a binding-dissociation of the EP complex. Each of these steps can be considered as completely independent reversible reactions, each with its own free energy change and associated kinetics.

The binding-dissociation reactions may be described by a dissociation constant, Kd. The intuitive interpretation of Kd is the concentration of substrate (S) where the enzyme (E) is half bound. Whereas the physical meaning of Kd is related to the free energy of binding as expressed in Equation (2),

$$\Delta G_{binding} = RT \cdot ln\, K_d$$

Equation (2)

where R is the ideal gas constant, T is absolute temperature, and $K_d$ is the dissociation constant. The sign is chosen so that a smaller $K_d$ corresponds to a more negative $\Delta G$. For example, at $K_d = 1$ mM, $\Delta G$ binding $\approx$ -17 kJ. Binding-dissociation is an equilibrium between: (i) association, governed by a rate constant $k_{on}$, and (ii) dissociation, governed by a rate constant $k_{off}$ (also called $k_1$ and $k_{-1}$), where $K_d$ is equal to $k_{off}/k_{on}$, as shown in FIG. 8 for the binding-dissociation of the ES complex. These binding-dissociation relationships between $K_d$, free energy $\Delta G$, rate constant $k_{on}$, and rate constant $k_{off}$ are exploited herein to freely interconvert between $K_d$, free energy $\Delta G$, on-rates, and off-rates.

Transition ES $\Leftrightarrow$ EP may be considered as an independent reaction, passing through a transition state. As shown in FIG. 9, there are two distinct energies to consider during transition: (i) the overall $\Delta G$ from ES to EP, and (ii) the activation energy $\Delta G \ddagger$ that is a barrier between these states. The relationship between the activation energy $\Delta G \ddagger$ and kfwd comes from the Arrhenius equation - Equation (3),

$$K = Ae^{-\Delta G\ddagger/RT}$$

Equation (3)

where $K$ is the rate constant, $Ae$ is a pre-exponential factor, $\Delta G \ddagger$ is activation energy, R is the ideal gas constant, and T is absolute temperature. The overall $\Delta G$ of the reaction can be converted to the rate constant k by the same equation as for $K_d$ above, which is equal to the ratio $k_{fwd}/k_{rev}$. Thus, as with the binding-dissociation reactions above, transition relationships between $K_d$, free energy $\Delta G$, rate constant $k_{fwd}$, and rate constant $k_{rev}$ are exploited herein to freely interconvert between free energy $\Delta G$ and forward and reverse rates.

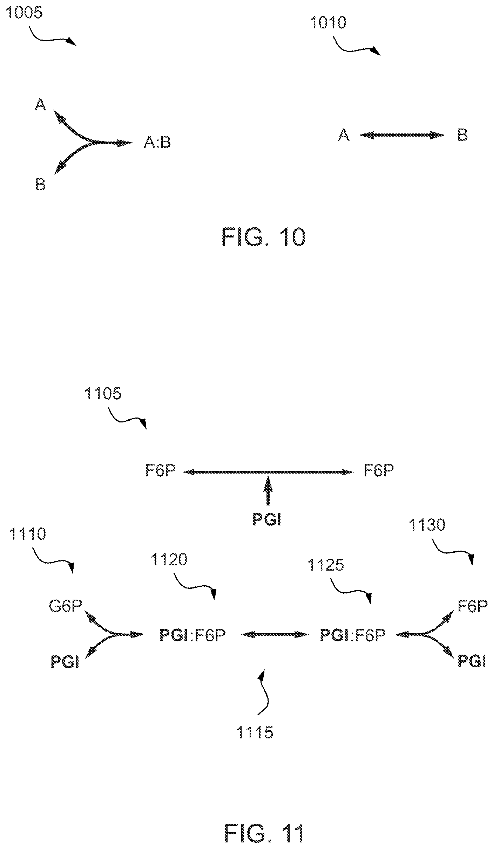

As shown in FIG. 10, the two basic components, a binding component 1005 and a transition component 1010, can be composed multiple ways to describe various enzymatic mechanisms of one or more reactions. The binding component 1005 comprises two molecules that associate or dissociate with some affinity. The transition component 1010 comprises the chemical conversion (forward and reverse) of one molecule to another through some transition state. Combinations of binding and transition components are very versatile in representing various enzymatic mechanisms of one or more reactions (a few common patterns cover most of the enzymatic mechanisms). For example, glucose-6-phosphate isomerase (GPI), alternatively known as phosphoglucoisomerase (PGI), is an enzyme that in humans is encoded by the GPI/PGI gene on chromosome 19. The enzyme has been identified as a moonlighting protein (a single protein that has multiple functions that are not due to gene fusions, multiple RNA splice variants or multiple proteolytic fragments) based on its ability to perform mechanistically distinct functions. In the cytoplasm, PGI functions as a glycolytic enzyme that interconverts glucose-6-phosphate (G6P) and fructose-6-phosphate (F6P). Extracellularly, PGI (also referred to as neuroleukin) functions as a neurotrophic factor that promotes survival of skeletal motor neurons and sensory neurons, and as a lymphokine that induces immunoglobulin secretion. As shown in FIG. 11, the intracellular enzymatic mechanism for PGI 1105 may be composed using a first binding component 1110 representing the association (e.g., binding) between PGI and G6P, a transition component 1115 representing the chemical conversion (forward and reverse) of the PGI:G6P complex 1120 to the PGI:F6P complex 1125, and a second binding component 1130 representing the dissociation (e.g., unbinding) between PGI and F6P.

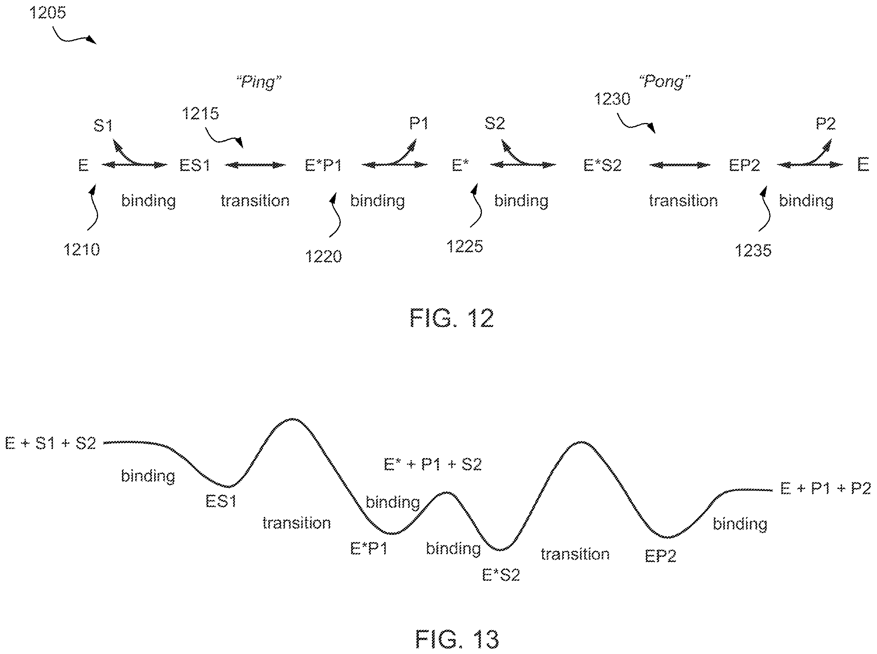

The standard example of an enzyme converting a single substrate to a single product in a reaction, however, is rare in practice. Most reactions involve two substrates and two products. One common enzymatic mechanism for such a reaction is called Ping-Pong Bi-Bi, as shown in FIG. 12. The Ping-Pong Bi-Bi mechanism 1205 may be composed using a first binding component 1210 representing the association of a first substrate (S1) (e.g., ATP) to an enzyme (E); and a first transition component 1215 representing the chemical conversion (forward and reverse) of the complex (E:S1) to a state that both converts the first substrate (S1) to a first product (P1) and modifies the enzyme (E) itself (e.g., by transfer of phosphate to an enzyme residue). The modification of the enzyme (E) is shown as (E*) in the complex (E*:P1). Ping-Pong Bi-Bi mechanism 1220 may be further composed using a second binding component 1220 representing the dissociation between the first product (P1) and the modified enzyme (E*); a third binding component 1225 representing the association of a second substrate (S2) to the modified enzyme (E*); and a second transition component 1230 representing the chemical conversion (forward and reverse) of the complex (E*:S2) to a state that both converts the second substrate (S2) to a second product (P2) and restores the modified enzyme (E*) to its original form (E) (e.g., by release of phosphate from the enzyme residue). The restoration of the enzyme (E) is shown as (E) in the complex (E:P2). Ping-Pong Bi-Bi mechanism 1220 may be further composed using a fourth binding component 1235 representing the dissociation between the second product (P2) and the enzyme (E). While the overall mechanism 1220 is more complicated, it can still be represented with a combination of binding and transition components, each with its own $\Delta G$ and rate constants. The whole process can be shown as a single path through a free energy space, as shown in FIG. 13.

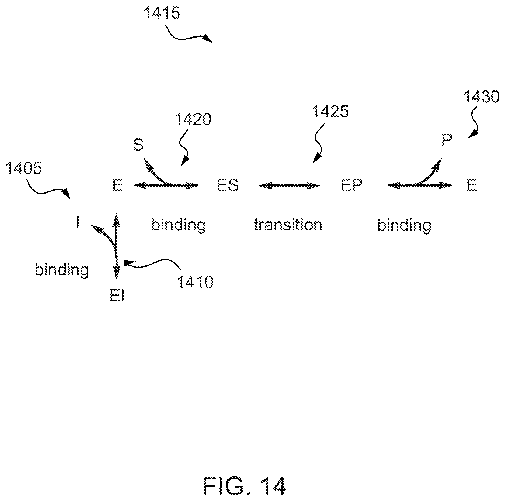

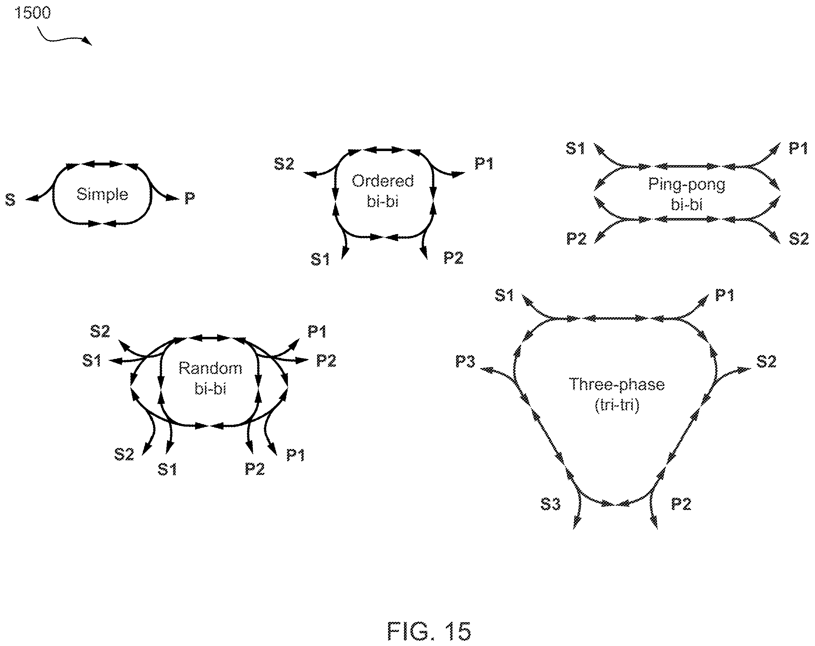

Another typical complexity is the binding of inhibitors which may also be represented with various combinations of binding and transition components. For example, basic competitive inhibition comprises the binding of an inhibitor at an active site of an enzyme, which sequesters the enzyme, and makes the sequestered enzyme completely unavailable to participate in the reaction. As shown in FIG. 14, the competitive inhibition mechanism 1405 may be composed using a first binding component 1410 representing the association of an inhibitor (I) to the active site of an enzyme (E) which sequesters some of the enzyme (E); and a simple enzyme mechanism 1415 comprising a second binding component 1420 representing the association (e.g., binding) between a substrate (S) and unsequestered or uninhibited enzyme (E), a transition component 1425 representing the chemical conversion (forward and reverse) of the E:S complex to the E:P complex, and a third binding component 1430 representing the dissociation (e.g., unbinding) between the product (P) and enzyme (E). FIG. 15 shows a potential catalog or knowledge base 1500 of enzymatic mechanisms with several levels of complexity composed using various combinations of binding and transition components.

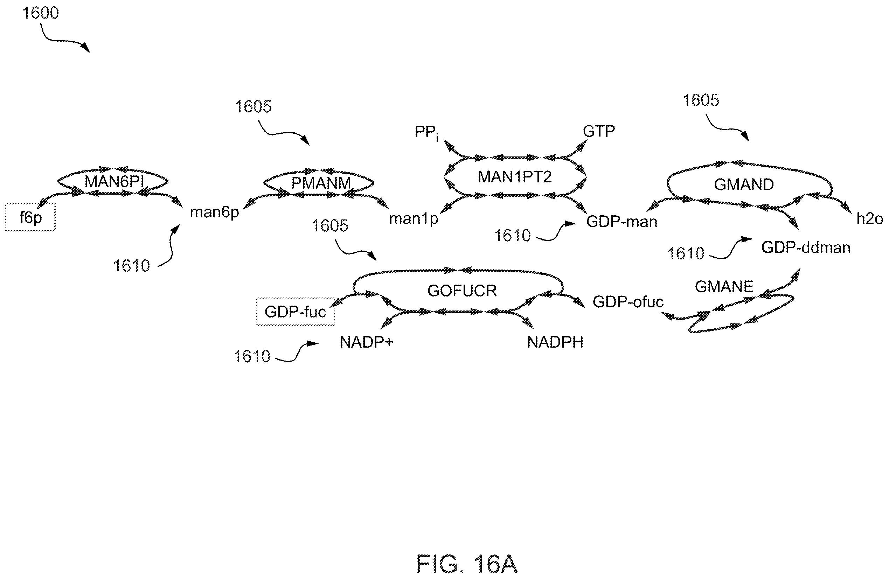

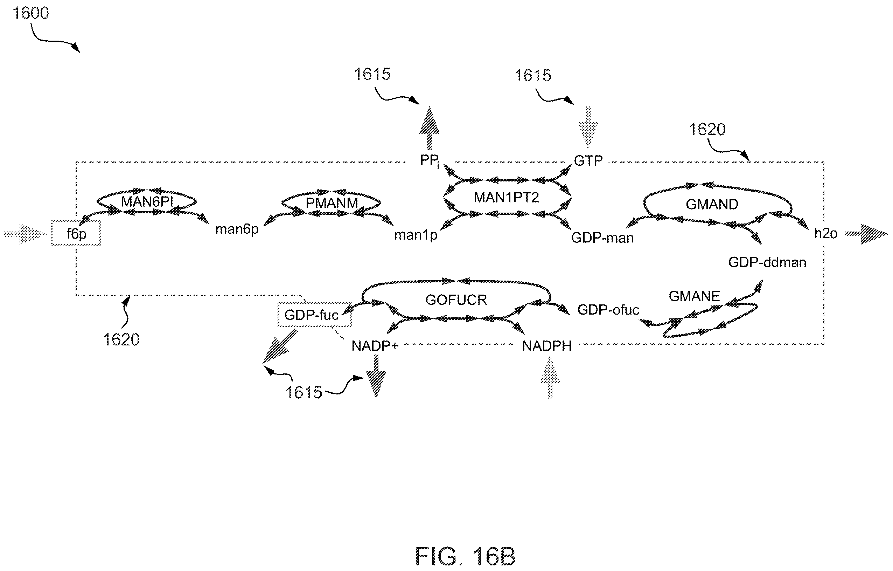

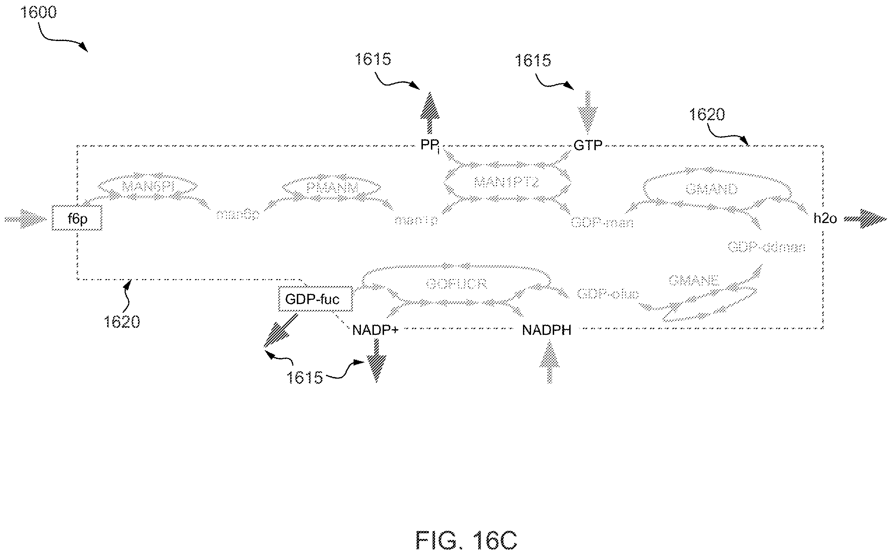

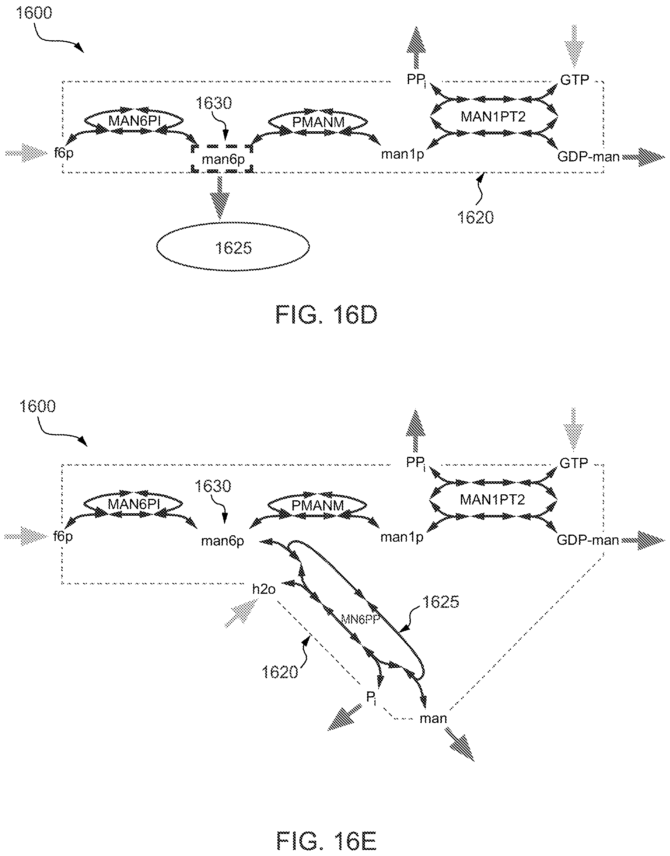

FIGS. 16A-16E show how an entire biological system, biochemical process, or biochemical pathway 1600 (e.g., the GDP-fucose pathway as shown) can be represented by multiple enzymatic mechanisms composed using various combinations of binding and transition components. The pathway 1600 is a set of reactions 1605 with shared molecules 1610 (e.g., metabolites). The molecules 1610 with outside connections 1615 that can be used to define the boundaries 1620 (see, e.g., FIG. 16B) of the pathway 1600. In some instances, a modeler (e.g., user or processor) decides which reactions 1605 to include in the pathway 1600, and where to put boundaries 1620. FIG. 16C shows the pathway 1600 from the outside once the boundaries 1620 are defined. FIGS 16D and 16E show that a modeler can take multiple actions towards reactions 1625 involving intermediate molecules 1630 including: (i) ignoring the reactions 1625 by making the intermediate molecules 1630 a boundary, (ii) adding the reactions 1625 to the pathway 1600 by extending the boundary 1620 to encompass the reactions, or (iii) removing the reactions 1625 from the pathway 1600 by contracting the boundary 1620 to exclude the reactions. Once the system, process, or pathway is constructed, an analytical solution is implemented that governs the behavior of each mechanism within the system, process, and/or pathway.

## IV. **Modeling and Simulation of Biochemical Reactions and Processes**

For modeling and simulation purposes described herein, there is a desire to predict a rate at which each reaction in a system is running in parallel given any condition. If it was possible to achieve a complete accounting of each of these rates, then it would be possible, in principle, to provide a complete detailed prediction of how the system will evolve over time (e.g., how concentrations of enzyme or product will change over time). Conventionally, the modeling and simulation of systems includes defining the detailed mechanisms for the system and attempting to find an analytical solution to produce one equation governing the overall behavior of the actions and/or components within the detailed mechanisms. However, an objective of the present disclosure is to model significantly larger and more complex systems (e.g., a whole cell), where such analytical solutions may not be possible, and where the approximations and assumptions conventionally used to find more local analytical solutions may not hold. Even well-established approximations such as Michaelis-Menten may not be appropriate for enzymes that are part of large, interconnected pathways.

Instead, various embodiments of the present disclosure are directed to techniques for modeling and simulating these systems, by: (i) systematically translating each component of a reaction into a set of rate equations to obtain a standard mathematical construct representing the individual binding or transition components regardless of the size of the biological system, and (ii) numerically integrating across the standard mathematical constructs using a system of ordinary differential equations to determine the contribution of each component to the rate of change of molecules with the mechanism or system. These techniques essentially provide a series of standardized mathematical constructs for each component that model the behavior of each component individually rather than providing a single algorithm that models the behavior of multiple components. Individually the standardized mathematical constructs are relatively easy to compute; and one of the few limitations to this approach is the amount of processing power available to compute multiple (tens, hundreds or thousands) standardized mathematical constructs in parallel to model or simulate the overall behavior of the mechanisms and system.

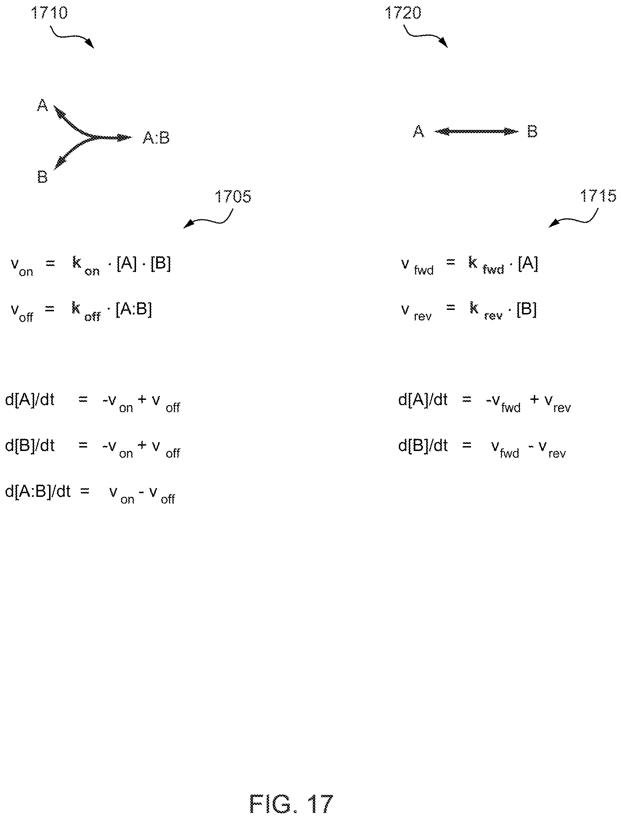

FIG. 17 shows a first standardized mathematical construct 1705 that may be used to model a single binding component 1710 (binding or dissociation) and a second standardized mathematical construct 1715 that may be used to model a single transition component 1720 (forward or reverse). Each rate equation of the standardized mathematical constructs has a simple form of one constant (kon, koff, kfwd, or krev) multiplied by a single concentration (e.g., [A], [B], or [A:B]) (a first order rate equation) or one constant (kon, koff, kfwd, or krev) multiplied by two concentrations (e.g., [A] and [B]) (second order rate equation). More specifically, the binding step of the binding component 1710 describes the association of one molecule [A] to one other molecule [B] to form a complex [A:B]. The forward rate von (left to right, binding) is a function of the concentrations of both molecules, i.e. a second-order term. The dissociation step of the binding component 1710 describes the dissociation of the complex [A:B] into molecule [A] and the other molecule [B]. The reverse rate voff (right to left, dissociation) is a function of the concentration of the complex [A:B], i.e., a first-order term. The transition step describes the chemical interconversion between two molecules [A] and [B]. The forward rate vfwd and the reverse rate vrev are both first-order terms, with the forward rate vfwd depending on the left-hand molecule [A], and the reverse rate vrev depending on the right-hand molecule [B]. Therefore, each component 1710; 1720 defines a single molecular event, contributing to a small number of terms in the ordinary differential equations describing the overall system. The power of this representation is that each type of component (binding or transition) is completely stereotypical, with kinetics described only in first and second order terms with respect to the system state. Nonetheless, the components 1710; 1720 can be composed into arbitrarily complex reaction mechanisms, following conventions commonly used in biochemistry to holistically model the overall behavior of systems. Advantageously, the total number of terms grows linearly as more components are added, and thus this technique scales easily in terms of size and complexity. Also advantageously, the standardized mathematical constructs are much simpler than conventional methods, which allows for reduced processor and memory constraints without a reduction in accuracy of modeling components.

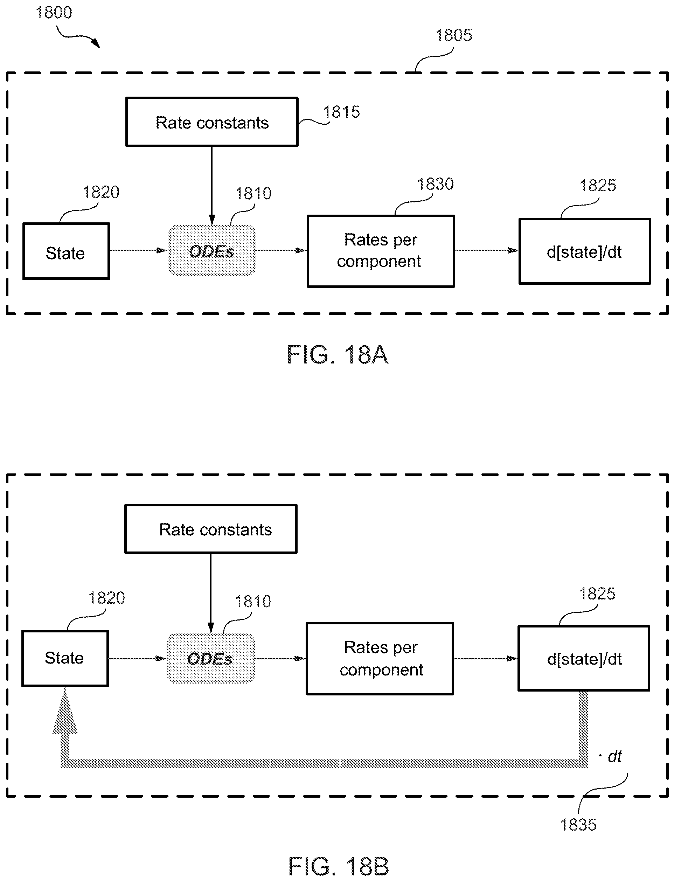

FIG. 18A shows a process flow 1800 for configuring components and sets of standardized mathematical constructs using an interaction system 1805 (e.g., interaction system 100 described with respect to FIG. 1) to simulate the behavior of a system (e.g., a system model such as the biological system model 200 described with respect to FIG. 2) by a simulation controller (e.g., simulation controller 300 described with respect to FIG. 3). Initially, a collection of related reactions, generally with shared molecules (e.g., enzymes, substrates or products), is defined for a kinetic pathway or mechanism(s) to be modeled, as described with respect to FIGS. 7-17. In some instances, higher-order attributes of the kinetic pathway or mechanism(s) may also be defined, such as which molecules are intermediates and which molecules are on the boundaries of the system. The configuration of the reactions and higher order attributes may comprise defining operations that translate relationships within the pathway or mechanism(s) into a system of ordinary differential equations 1810 (ODEs). The interaction system 1805 manages all of the operations to translate the kinetic pathway or mechanism(s) in to the system of ordinary differential equations 1810.

For example, each reaction of the kinetic pathway or mechanism(s) may be defined as an instance of a reaction class or object. Each instance of the reaction class or object may be defined via a list of instances of components, i.e., the component steps (binding, dissociation, and transition) of the overall reaction, and associated sets of standardized mathematical constructs that govern behavior of the components. The sets of standardized mathematical constructs defining the forward and reverse rates of binding, dissociation, and transition steps for the components in terms of first-order and second-order terms using concentrations of molecules in the system and forward and reverse rate constants 1815. The reaction class or object tracks a list of substrates, a list of products, and the enzyme that catalyzes the reaction. In some instances, the reaction class or object defines all of the intermediate forms of the enzyme, generally comprising the base enzyme bound to one or more substrates or products, as appropriate to the specific mechanism being modeled. Each component (binding or transition) within the list of instances of components defines a single molecular event, contributing to a small number of terms in a system of ordinary differential equations 1810 describing the overall system. A utility class or object may be used to map the variables of the kinetic pathway or mechanism(s) (e.g., concentrations of molecules such as concentration of enzyme, concentration of product, concentration of substrate, concentration of intermediates, etc.) calculated within the system of ordinary differential equations 1810 to and from numerical indices in an array of any number of dimensions such as a current state vector 1820 and a net rate of change vector.

The current state vector 1820 and the forward and reverse rate constants 1815 are input into the system of ordinary differential equations 1810. The system of ordinary differential equations 1810 may be represented by a single matrix built piecewise indicating the contribution of each component to the rate of change of each molecule 1825 of the system. The ordinary differential equations 1810 utilize the sets of standardized mathematical constructs to calculate the forward and reverse rates for each component within the instances of the reaction class or object as a function of the current state. The forward and reverse rates of each component are calculated independently, and then the forward and reverse rates for each component are summed to obtain the net rate of each step of the reaction 1830 (e.g., binding, dissociation, or transition). Each calculation follows mass-action kinetics, with terms that are either first-order or second-order, depending on the type of component. The ordinary differential equations 1815 numerically integrate across the set of standard mathematical constructs to determine the contribution of each component to the net rate of change of the molecules 1825 within the system based on the net rate of each step of the reaction 1830. The net rate of change of the molecules 1825 may be stored and maintained on a net rate of change vector.

FIG. 18B shows that if the model for the system is to be simulated over time, then the net rate of change 1825 for each molecule can be applied iteratively over some unit of time 1835 (i.e., a variable time step determined by the system based on concentrations of molecules in the system and a total time that the system is to evolve over (second, minutes, hours, etc.)) back to the current state vector 1820, which updates the current state vector 1820 to reflect the change of state of each molecule over the unit of time 1835. Consequently, the system of ordinary differential equations 1815 perform a numerical integration over the sets of standardized mathematical constructs for each unit of time 1835 that pulls together all the calculations into an instantaneous rate of change of all molecules in the system, which can be used to update a continuous number representing the concentration of each molecule on the current state vector 1820.

The standardized mathematical constructs (forward and reverse rates - von, voff, vfwd, vrev) of each component are governed by the rate constants 1815 (kon, koff, kfwd, or krev). More specifically, there is a forward rate constant (e.g., kon or kfwd) and a reverse rate constant (e.g., koff or krev) of each component, and these forwards and reverse rate constants 1815 are the parameters which must be supplied to build the model of the kinetic pathway or mechanism(s) for a system. The parametrization of the model with these forward and reverse rate constants 1815 can be error prone if the rate constants are treated individually because there is missing information or knowledge about how thermodynamics works in each of these mechanisms. Morevoer, the rate constants 1815 (kon, koff, kfwd, or krev) cannot be looked up in published material and cannot be determined experimentally. Thus, techniques are required for representing these forward and reverse rate constants 1815 so that the rate constants 1815 can be used in the system of ordinary differential equations 1815 to simulate the system.

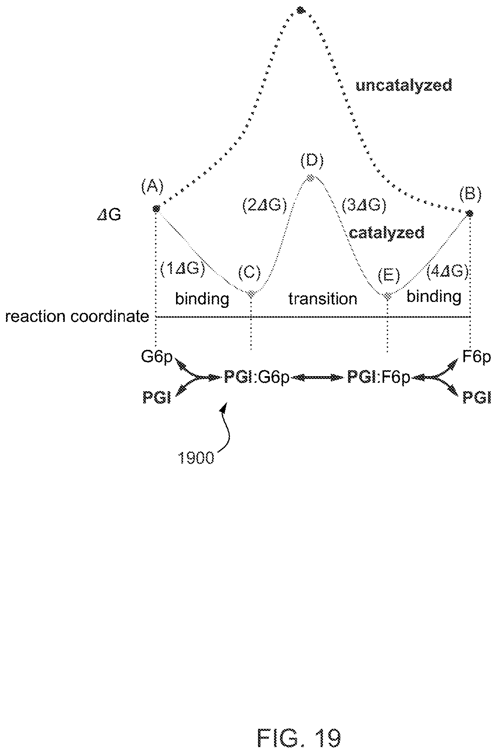

In various instances, techniques are disclosed for predicting these forward and reverse rate constants using free energy transitions ($\Delta G$) for a given binding component or transition component. As shown in FIG. 19, the free energy states for a typical enzyme reaction 1900 comprise a first free energy state (A) for E+S and a second free energy state (B) for E+P, which are defined and unchangeable. Additionally, there are three free energy states between E+S and E+P (described herein as "waypoints" between E+S and E+P) comprising a third free energy state (C) for ES, a fourth free energy state (D) for the transition state between ES and EP, and a fifth free energy state (F) for EP, which are changeable to define a path from substrate to product (e.g., uncatalyzed path versus catalyzed path). Between these five free energy states there are free energy transitions ($\Delta G$) that can be used to predict the forward and reverse rate constants (kon, koff, kfwd, or krev). The free energy transitions ($\Delta G$) include: a first free energy transition ($1\Delta G$) from E+S to ES, a second free energy transition ($2\Delta G$) from ES to the transition state between ES and EP, third free energy transition ($3\Delta G$) from the transition state between ES and EP to EP, and a fourth free energy transition ($4\Delta G$) from EP to E+P.

The thermodynamics are given as input, represented as a free energy profile comprising a sequence of free energy transitions ($\Delta G$) over the course of a component reaction. Each component may be treated differently for predicting the forward and reverse rate constants using free energy transitions ($\Delta G$). In general the conversion of the sequence of free energy transitions ($\Delta G$) to forward and reverse rate constants takes the form of the Arrhenius equation - Equation (4),

$$K = Ae^{+/-\Delta G\ddagger/RT}$$

Equation (4)

where $k$ is the rate constant, $Ae$ is a pre-exponential factor, $\Delta G\ddagger$ is activation energy, R is the ideal gas constant, and T is absolute temperature. The exponential factors +/-1/RT and pre-exponential factor $Ae$ may be built within the system of ordinary differential equations by the piecewise contribution of each component via calls to their respective exponential factors and pre-exponential factor methods with applicable reindexing.

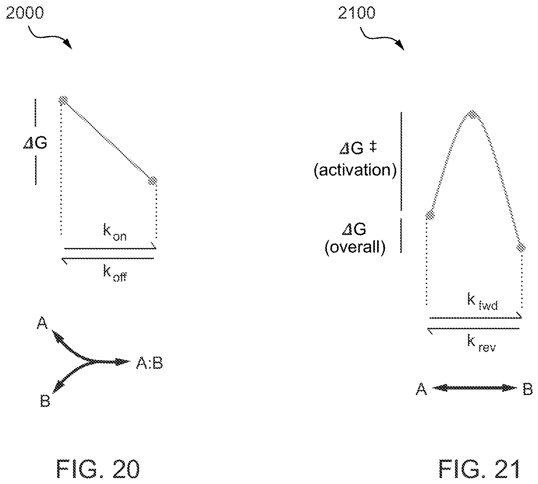

As shown in FIG. 20, a binding (or dissociation) step 2000 for two molecules can be described as a relationship or a ratio of concentrations for the two molecules [A:B]/[A][B] at an end point. If the molecules have a very strong affinity for one another, then there will be much more bound molecules [A:B] as compared to the free molecules [A] and [B], which corresponds to a negative $\Delta G$ with a steep slope within the free energy landscape (a faster rate of binding). Alternatively, if the molecules have a weaker affinity for one another, then there will be a slightly different ratio (e.g., less bound molecules [A:B] and a more free molecules [A] and [B]), which corresponds to a negative $\Delta G$ with a gentle slope within the free energy landscape ( a slower rate of binding). For a component at equilibrium this relationship of the molecules within the free energy landscape can be expressed by the first law of thermodynamics - Equation (5):

$$K_{binding} = [A:B]_{eq}/[A]_{eq}[B]_{eq} = e^{-\Delta G/RT}$$

Equation (5)

where $K_{binding}$ (or the opposite $K_d$ which is the dissociation constant) is the overall rate of the binding or dissociation step [A:B] is the concentration of complex A:B at equilibrium, [A] is the concentration of A at equilibrium, [B] is the concentration of B at equilibrium, e is a pre-exponential factor, $\Delta G$ is overall energy, R is the ideal gas constant, and T is absolute temperature. At any time, the on-rate or forward rate of the component can be expressed as $k_{on}[A][B]$ and the off-rate or reverse rate of the component can be expressed as $k_{off}[A:B]$, and at equilibrium the on-rate = the off-rate, and thus $K_{binding}$ may be expressed as a ratio of $k_{on}[A][B]$ to $k_{off}[A:B]$ (or as $k_{on}/k_{off}$). In certain instances, $k_{on}$ may be treated as a constant (i.e., an assumption) limited by diffusion rate and collision frequency, and thus $k_{off}$ may be expressed by Equation (6):

$$k_{off} = k_{on}/K_{binding} = k_{on}e^{+\Delta G/RT}$$

Equation (6)

where $k_{on}$ is the on-rate or forward rate of the component, $K_{binding}$ is the overall rate of the binding or dissociation step, e is a pre-exponential factor, $\Delta G$ is overall energy, i.e. negative $\Delta G$ means lower off-rate, R is the ideal gas constant, and T is absolute temperature.

As shown in FIG. 21, a transition step 2100 for two molecules goes through an internal transition state that serves as a barrier within the free energy landscape to the rate of transition from [A] to [B] or from [B] to [A], and thus a relationship exists between a height of the barrier and the rate of the transition step (forward and reverse rates). For a component at equilibrium this relationship of the molecules within the free energy landscape can be expressed by the first law of thermodynamics - Equation (5) where Ktransition = [A]eq/[B]eq = kfwd/krev = e-$\Delta$G/RT. In certain instances, kfwd can be expressed by the Arrhenius Equation - Equation (4) where k is the rate constant for kfwd, Ae is a pre-exponential factor, $\Delta$G$\ddagger$ is activation energy, R is the ideal gas constant, and T is absolute temperature. Thus, krev can be expressed by Equation (7):

$$k_{rev} = k_{fwd}/K_{transition} = Ae^{(\Delta G - \Delta G\ddagger)/RT}$$

Equation (7)

where $k_{rev}$ is the reverse rate of the component, $k_{fwd}$ is the forward rate of the component, $K_{transition}$ is the overall rate of the transition step, *Ae* is a pre-exponential factor, $\Delta$G is overall energy, $\Delta$G$\ddagger$ is activation energy, R is the ideal gas constant, and T is absolute temperature. Consequently, standardized mathematical constructs ($v_{on}$, $v_{off}$, $v_{fwd}$, $v_{rev}$) can be implemented that govern the forward and reverse rates of each component step, and the forward and reverse rate constants ($k_{on}$, $k_{off}$, $k_{fwd}$, or $k_{rev}$) driving these standardized mathematical constructs can be calculated from the free energy profile for each component step. The total number of terms (first-order terms and second-order terms) for the system grows linearly as more components are added to model the kinetic pathway or mechanism(s) of the system.

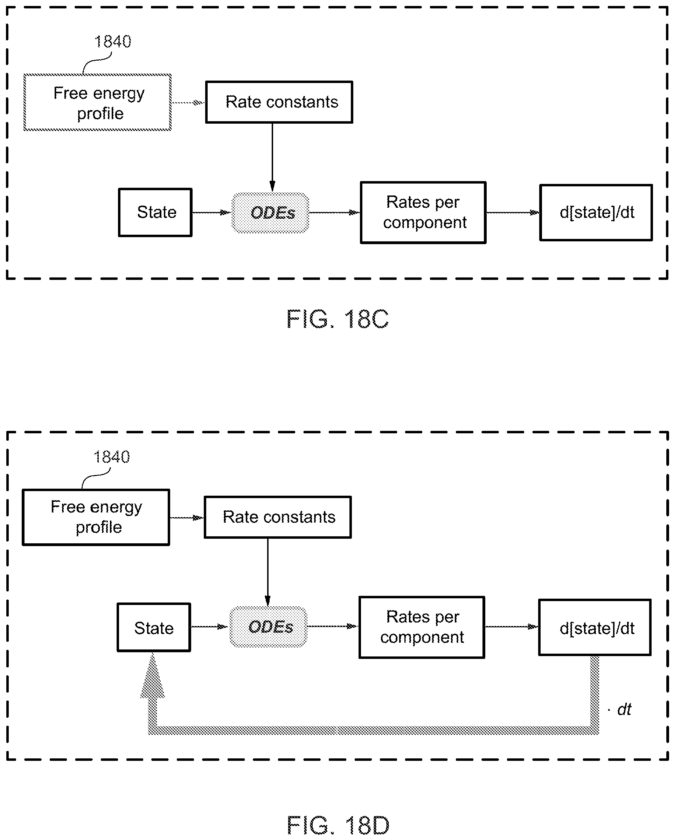

Using the techniques described herein for predicting the forward and reverse rate constants using free energy transitions ($\Delta$G), the process 1800 can be expanded to start with free energy profiles 1840 for each component from which it is possible to determine the forward and reverse rate constants, as shown in FIGS. 18C and 18D. However, there are a number of parameters for the free energy profiles 1840 (i.e., $\Delta$G overall energy for each binding step; and $\Delta$G$\ddagger$ activation energy and $\Delta$G overall energy for each transition step) that may not be known in some instances. Thus, additional techniques are disclosed herein for obtaining values for the free energy profiles 1840 in those instances to parameterize the model.

## V. <u>Using Machine Learning to Fit Kinetic Parameters to Known Data</u>

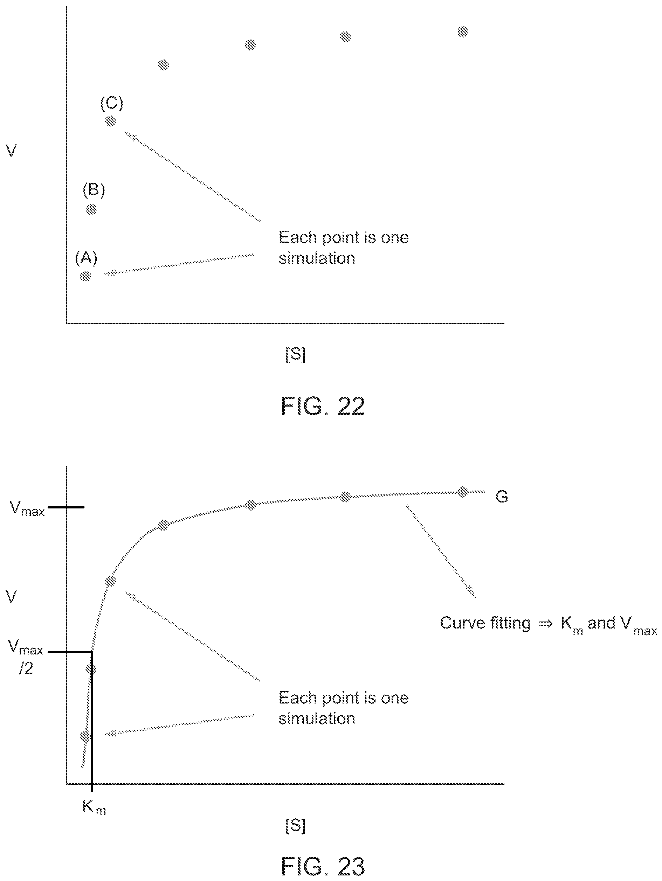

As shown in FIG. 22, if a concentration range for a substrate [S] (x-axis) is input into a model (as generated in accordance with FIGS. 1-17), then using the processes described with respect to FIGS 18A-18D it is possible to determine the rate of a reaction V for each simulation when the model is run in silico. Each point on the graph represents a single simulation where the energy profile and forward and reverse rate constants are held invariant or constant and the concentration of substrate (current state vector) is varied over time (e.g., increased). For example, the model may be run in silico to obtain a first data point (A) representing a first simulation run with a first substrate concentration [S], a second data point (B) representing a second simulation run with a second substrate concentration [S+n], a third data point (C) representing a third simulation run with a third substrate concentration [S+n+m], and more of the same iteratively until a relationship is developed between the concentration of substrate [S] versus the rate of the reaction V.

As shown in FIG. 23, once enough data points are captured for the concentration range for the substrate [S], a best-fit line curve can be generated by the interaction system to connect the data points and graph the reaction rate V as a function of substrate concentration [S]. In some instances, the graph obtained will resemble the line G where the V will increase rapidly at low substrate concentrations [S], then level off to a flat plateau at high substrate concentrations [S]. The plateau occurs because the enzyme is saturated, meaning that all available enzyme molecules are already tied up processing substrates. Any additional substrate molecules will simply have to wait around until another enzyme becomes available, so the rate of reaction (amount of product produced per unit time) is limited by the concentration of enzyme. This maximum rate of reaction is characteristic of a particular enzyme at a particular concentration and is known as the maximum velocity Vmax and is typically known for most enzymes (i.e., the Y-value (initial rate of reaction value) at which the graph above plateaus). The substrate concentration that gives a rate that is halfway to Vmax is called the Km (Michaelis constant), and is a useful measure of how quickly reaction rate increases with substrate concentration. Km is also a measure of an enzyme's affinity for (tendency to bind to) its substrate. A lower Km corresponds to a higher affinity for the substrate, while a higher Km corresponds to a lower affinity for the substrate. Unlike Vmax, which depends on enzyme concentration, Km is always the same for a particular enzyme characterizing a given reaction and is typically known for most enzymes.

The ability to develop a relationship between the concentration of substrate [S] versus the rate of the reaction V based on free energy profiles to obtain values for Vmax and Km forms the basis of a design for a machine learning approach in accordance with aspects of the present disclosure. In various embodiments, the machine learning approach comprises: (i) defining a system to be simulated in terms of binding and transition steps, (ii) learning $\Delta G$ overall energy for each binding step and $\Delta G\ddagger$ activation energy and $\Delta G$ overall energy for each transition step subject to constraints on their sum across each overall reaction, (iii) translating, at each evaluation of the system, the various parameters to kon, koff, kfwd, or krev rate constants, (iv) determining steady state concentrations of all molecules including intermediates and corresponding fluxes by solving a resulting system of ordinary differential equations, and (v) evaluating loss against known Vmax and Km parameters.

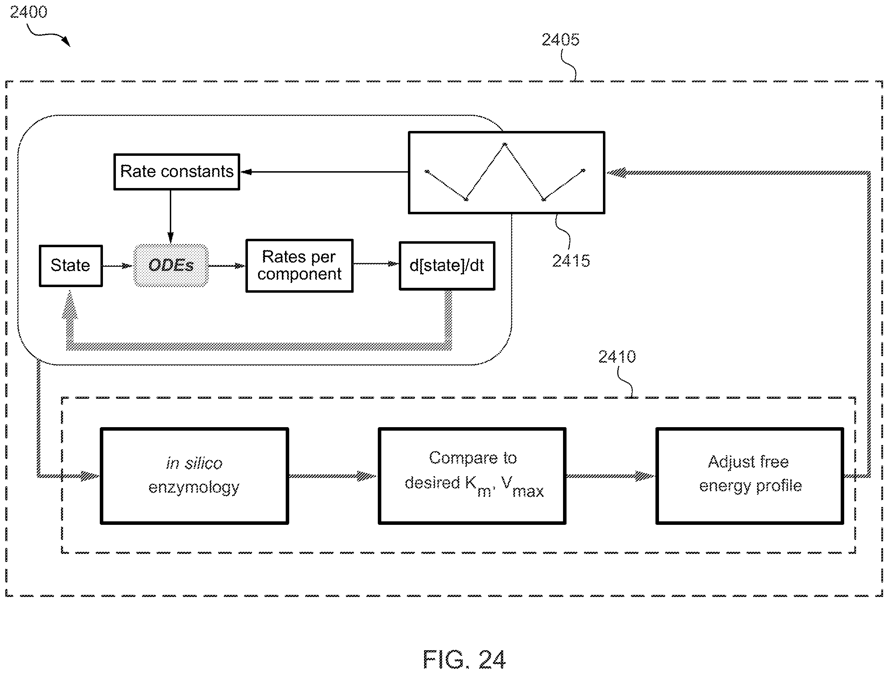

FIG. 24 shows a process flow 2400 for configuring components and sets of standardized mathematical constructs using an interaction system 2405 (e.g., interaction system 100 described with respect to FIG. 1) to simulate the overall behavior of a system (e.g., a system model such as the biological system model 200 described with respect to FIG. 2) by a simulation controller (e.g., simulation controller 300 described with respect to FIG. 3) implementing a prediction model 2410. Initially, a collection of related reactions, generally with shared molecules (e.g., substrates or products), is defined for a kinetic pathway or mechanism(s) to be modeled, as described with respect to FIGS. 10-17, and an energy profile 2415 is obtained for each reaction. The defining the collection of reactions for the system to be modeled may comprise defining the expected behavior for each reaction. The expected behavior may be defined based on the molecules used within each component step. For example, Vmax and Km, which define the expected behavior for given molecules, can be obtained from a knowledge bank or data storage and used to define the behavior of each component step that includes the given molecules. The energy profiles 2415 comprise the free energy state $\Delta G$ for each component step, e.g., $\Delta G$ overall energy for each binding step and $\Delta G\ddagger$ activation energy and $\Delta G$ overall energy for each transition step. The energy profiles 2415 may be initially obtained arbitrarily (e.g., selected by a user or the integration system randomly or without reason), rationally (e.g., selected by a user or the integration system with some reasoning), explicitly (e.g., selected by a user or the integration system from a library of energy profiles developed for the same binding or transition step), or a combination thereof.

Using the processes described with respect to FIGS 18A-18D it is possible to calculate the forward and reverse rate constants (kon, koff, kfwd, or krev) based on the obtained energy profiles, calculate the forward and reverse rates of each component independently using the forward and reverse rate constants (kon, koff, kfwd, or krev) and the standardized mathematical constructs (von, voff, vfwd, vrev), and then the forward and reverse rates for each component are summed to obtain the net rate of each step of the reaction (e.g., binding, dissociation, or transition). The system of ordinary differential equations numerically integrates across the set of standard mathematical constructs to determine the contribution of each component to the net rate of change of each molecule within the system based on the net rate of each step of the reaction. The in silico behavior of the system derived from the current state vector, the net rate of each step of the reaction, and the net rate of change of each molecule is provided to the prediction model 2410 as input data for determining the Vmax and Km of the current simulation for each reaction, comparing the Vmax and Km for the in silico behavior of each reaction to the known Vmax and Km for the expected behavior of each reaction, and predicting a new energy profile based on a fitting of the in silico behavior to the expected behavior, and adjusting energy profiles 2415 accordingly.

The prediction model 2410 can be a machine-learning model, such as a neural network, a convolutional neural network ("CNN"), e.g. an inception neural network, a residual neural network ("Resnet") or NASNET provided by GOOGLE LLC from MOUNTAIN VIEW, CALIFORNIA, or a recurrent neural network, e.g., long short-term memory ("LSTM") models or gated recurrent units ("GRUs") models. The prediction model 2410 can also be any other suitable machine-learning model trained to predict energy profiles for a reaction, such as a support vector machine, decision tree, a regression model, coarse-grained models, normal mode analysis, or Markov state models, random forest classifier, a three-dimensional CNN ("3DCNN"), a dynamic time warping ("DTW") technique, a hidden Markov model ("HMM"), etc., or combinations of one or more of such techniques-e.g., CNN-HMM or MCNN (Multi-Scale Convolutional Neural Network).

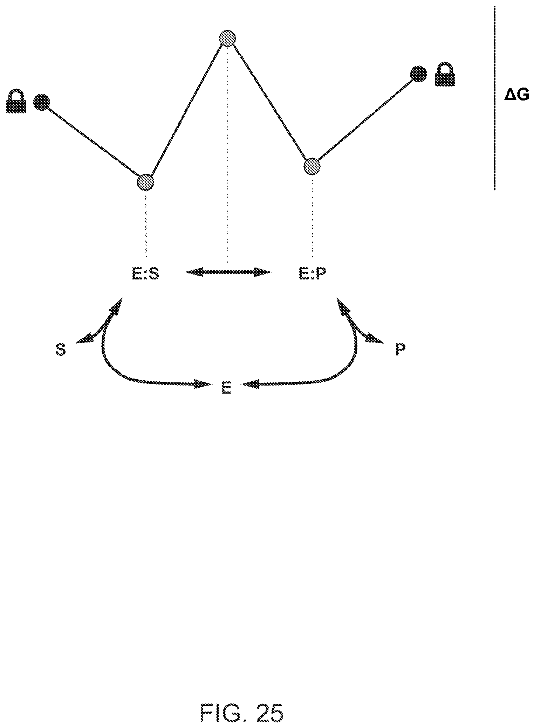

To train the prediction model 2410, training samples are obtained or generated. The training samples can include a current state vector, a net rate of each step of the reaction, and a net rate of change of each molecule for various component steps, and optional labels corresponding to energy profile parameters such as $\Delta G$ overall energy for each binding step and $\Delta G\ddagger$ activation energy and $\Delta G$ overall energy for each transition step (including: a first free energy transition ($1\Delta G$) from E+S to ES, a second free energy transition ($2\Delta G$) from ES to the transition state between ES and EP, third free energy transition ($3\Delta G$) from the transition state between ES and EP to EP, and/or a fourth free energy transition ($4\Delta G$) from EP to E+P). In some instances, the training process includes iterative operations to find a set of parameters for the prediction model 2410 that minimizes a loss function for the prediction models 2410. Each iteration can involve finding a set of parameters for the prediction model 2410 so that the value of the loss function using the set of parameters is smaller than the value of the loss function using another set of parameters in a previous iteration. The set of parameters may comprise the waypoints or free energy states between E+S and E+P comprising a free energy state (A) for ES, a free energy state (B) for the transition state between ES and EP, and a free energy state (C) for EP, which are changeable to define a path from substrate to product (e.g., uncatalyzed path versus catalyzed path), as shown in FIG. 25. The loss function can be constructed to measure the difference between the outputs predicted using the prediction models 2410 (e.g., $\Delta G$ overall energy for each binding step and $\Delta G\ddagger$ activation energy and $\Delta G$ overall energy for each transition step) and the optional labels contained in the training samples. In some instances, the loss function may be constructed to measure this difference based on the comparison between the determined Vmax and Km of the current simulation using a given or predicted energy profile and the known Vmax and Km for the expected behavior of a reaction. Once the set of parameters are identified, the prediction model 2410 has been trained and can be tested, validated, and/or utilized for prediction in simulations as designed.

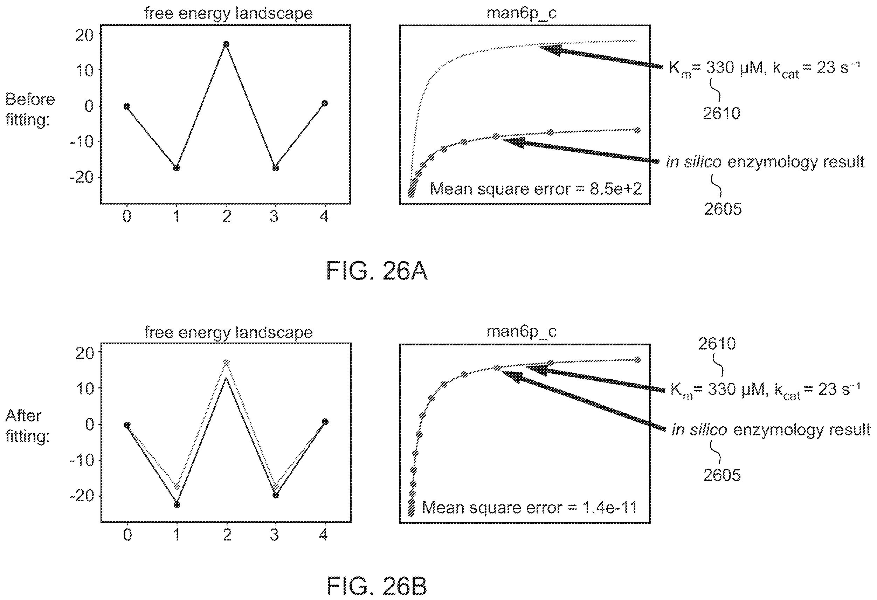

In some instances, prior to a fitting and prediction by the prediction model 2410, the simulation of a model for a system may be run iteratively to obtain in silico behavior of each reaction of the collection of reactions based on an initial energy profile and derive the reaction rate of each reaction as a function of substrate concentration. As shown in FIG. 26A, the in silico behavior 2605 of a reaction determined based on the initial energy profile obtained prior to a fitting and prediction by the prediction model 2410 may be far from meeting the expected behavior 2610 of the reaction based on a comparison between the Vmax and Km for the in silico behavior of the reaction and the known Vmax and Km for the expected behavior of the reaction. Consequently, the in silico behavior 2605 including the current state vector, the net rate of each step of the reaction, and the net rate of change of each molecule may be provided to the prediction model 2410 as input data for predicting a new energy profile for the reaction based on the fitting of the in silico behavior to the expected behavior and the loss function. The predicting may comprise learning a set of parameters including the waypoints or free energy states between E+S and E+P, which are changeable to define a path from substrate to product to ultimately fit the in silico behavior to the expected behavior. The predicting may further comprise outputting a new energy profile comprising a $\Delta G$ overall energy for each binding step and $\Delta G\ddagger$ activation energy and $\Delta G$ overall energy for each transition step (e.g., including: a first free energy transition (1$\Delta G$) from E+S to ES, a second free energy transition (2$\Delta G$) from ES to the transition state between ES and EP, third free energy transition (3$\Delta G$) from the transition state between ES and EP to EP, and/or a fourth free energy transition (4$\Delta G$) from EP to E+P).

After a fitting and prediction by the prediction model 2410, the simulation of the model for the system may again be run iteratively to obtain in silico behavior of each reaction of the collection of reactions based on the new energy profile and derive the reaction rate of each reaction as a function of substrate concentration. As shown in FIG. 26B, the in silico behavior 2605 of a reaction determined based on the new energy profile obtained after the fitting and prediction by the prediction model 2410 may be substantially closer to meeting the expected behavior 2610 of the reaction based on a comparison between the Vmax and Km for the in silico behavior of the reaction and the known Vmax and Km for the expected behavior of the reaction. In some instances, if the in silico behavior still does not meet the expected behavior of the reaction, then the in silico behavior 2605 including the current state vector, the net rate of each step of the reaction, and the net rate of change of each molecule may be provided to the prediction model 2410 as input data for predicting an updated energy profile for the reaction based on the fitting of the in silico behavior to the expected behavior and the loss function. In some instances, whether the in silico behavior meets the expected behavior of the reaction may be determined when the Vmax and Km for the in silico behavior of the reaction is equal to or substantially equal to the known Vmax and Km for the expected behavior. The training, predicting, and fitting processes may be performed iteratively until the in silico behavior meets the expected behavior.

## VI. **Techniques for Modeling Kinetics**

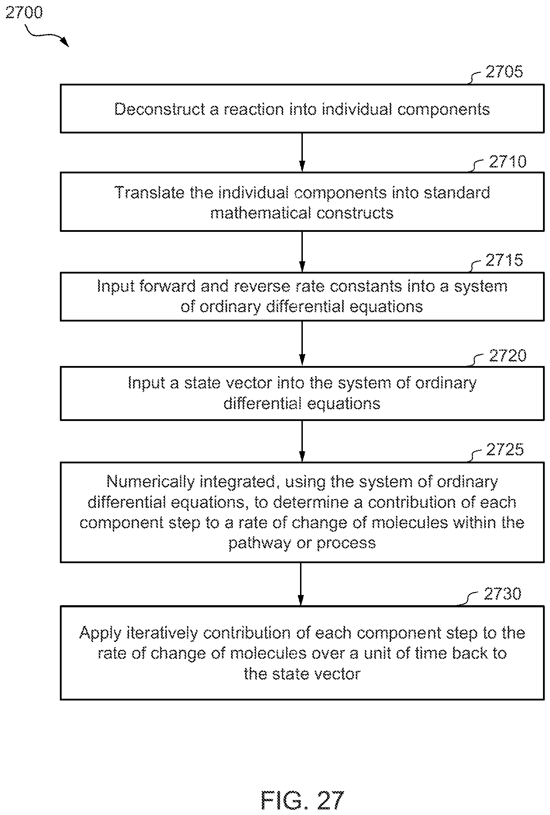

FIG. 27 is a simplified flow chart illustrating an example of process 2700 for modeling a reaction pathway, process, or system using an interaction system (e.g., interaction system 100 described with respect to FIG. 1) to simulate behavior of a system (e.g., a system model such as the biological system model 200 described with respect to FIG. 2) by a simulation controller (e.g., simulation controller 300 described with respect to FIG. 3) according to various embodiments. Process 2700 begins at block 2705, at which a reaction is deconstructed into a plurality of component steps. The reaction may be part of a pathway or process in a system to be modeled. At block 2710, each component step of the plurality of component steps is translated into a set of rate equations to obtain a standard mathematical construct (i.e., a mathematical model) representing each component step. The set of rate equations comprise a forward rate equation and a reverse rate equation, and each forward rate equation and each reverse rate equation is a first-order rate equation or a second-order rate equation. In some instances, the plurality of component steps comprise at least two binding component steps and at least one transition component step Each binding component step of the at least two binding component steps comprises: (i) binding between an enzyme and a substrate, or (ii) dissociation of an enzyme from a product, and each transition component step of the at least one transition component step comprises a chemical conversion of an enzyme:substrate complex to a product:enzyme complex. The forward rate equations may have a form of a forward rate constant kon or kfwd multiplied by a single concentration or two concentrations, and the reverse rate equations may have a form of a reverse rate constant koff or krev multiplied by a single concentration or two concentrations. The forward rate constants and the reverse rate constants may be derived from an energy profile for the reaction.

At block 2715, the forward rate constants and the reverse rate constants are input into a system of ordinary differential equations. At block 2720, a state vector comprising concentrations for at least some of the molecules of the reaction pathway, process, or system are input into the system of ordinary differential equations. At block 2725, the standard mathematical constructs are numerically integrated using a system of ordinary differential equations to determine a contribution of each component step of the plurality of component steps to a rate of change of molecules within the pathway or process. The integrating may comprise determining forward and reverse rates for each component step of the plurality of component steps based on the standard mathematical construct representing each component step, and summing the forward and reverse rates for each component step to obtain a net rate of each component step of the reaction. The contribution of each component step to the rate of change of molecules within the pathway or process is determined based on the net rate of each component step of the reaction. In some instances, the system of ordinary differential equations is represented by a single matrix built piecewise indicating the contribution of each component step to the rate of change of the molecules of within the pathway or process.

At block 2730, the contribution of each component step to the rate of change of the molecules within the pathway or process is applied iteratively over a unit of time back to the state vector. For example, after determining the contribution of each component step to the rate of change of the molecules within the pathway or process, a cross-module result synthesizor can access these module-specific results (from one or more module-specific results data stores or direct data availing) and synthesize the results to update high-level data such as a state vector (e.g., stored in a high-level metabolite data store). The state vector may then be used as input to simulate states and reactions of modules configured by an integrated development environment (e.g., the interaction system 100 described with respect to FIG. 1) and/or used by a simulator (e.g., module-specific simulation controller 500 described with respect to FIG. 5) to generate metabolite time-course data according to various embodiments.

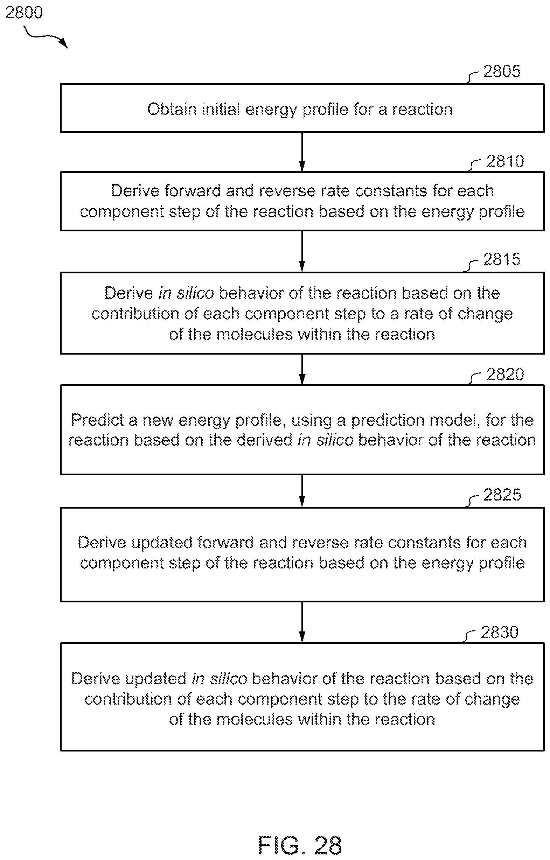

FIG. 28 is a simplified flow chart illustrating an example process 2800 for predicting parameters and parameterizing a model for a reaction using a protein development platform and a machine-learning modeling system and technique (e.g., the computing environment 100 and machine-learning modeling system and technique 200, 300, 400 described with respect to FIGS. 1, 2A-2H, 3, and 4) according to various embodiments. Process 2800 begins at block 2805, at which an initial energy profile for a reaction is obtained. The reaction may be part of a pathway or process in a system to be modeled (see, e.g., process 2700). At block 2810, forward and reverse rate constants are derived for each component step of a plurality of component steps representing the reaction based on the initial energy profile. Each component step is represented by a standard mathematical construct (i.e., a mathematical model) comprising a set of forward and reverse rate equations that apply the forward and reverse rate constants to one or more concentrations of molecules, respectively. In some instances, the plurality of component steps comprise at least two binding component steps and at least one transition component step. Each binding component step of the at least two binding component steps may comprise: (i) binding between an enzyme and a substrate, or (ii) dissociation of an enzyme from a product. Futher, each transition component step of the at least one transition component step may comprise a chemical conversion of an enzyme: substrate complex to a product:enzyme complex. The binding between the substrate and the enzyme contributes a first $\Delta G$ overall energy to the energy profile, the dissociation of the enzyme from the product contributes a second $\Delta G$ overall energy to the energy profile, and the chemical conversion of the enzyme:substrate complex to the product:enzyme complex contributes a $\Delta G\ddagger$ activation energy and a third $\Delta G$ overall energy to the energy profile. In some instances, each forward rate equation has a form of a forward rate constant $k_{on}$ or $k_{fwd}$ multiplied by a single concentration or two concentrations. Each reverse rate equation may have a form of a reverse rate constant $k_{off}$ or $k_{rev}$ multiplied by a single concentration or two concentrations.

At block 2815, a in silico behavior of the reaction is derived based on the contribution of each component step of the plurality of component steps to a rate of change of the molecules within the reaction. The contribution of each component may be determined from the standard mathematical construct applying the forward and reverse rate constants to the one or more concentrations of molecules, respectively. In some instances, the deriving the in silico behavior of the reaction comprises numerically integrating across the standard mathematical constructs using a system of ordinary differential equations to determine the contribution of each component step to the rate of change of molecules within the reaction. The system of ordinary differential equations may be represented by a single matrix built piecewise indicating the contribution of each component step to the rate of change of the molecules within the reaction. In some instances, the contribution of each component step to the rate of change of the molecules within the pathway or process is applied iteratively over a unit of time back to the state vector.

At block 2820, a new energy profile is predicted, using a prediction model, for the reaction based on the derived in silico behavior of the reaction. The predicting may comprise learning a set of parameters including waypoints or free energy states, which are changeable to define a path of the reaction from substrate to product to fit the in silico behavior of the reaction to an expected behavior of the reaction. In some instances, the prediction model comprises a loss function constructed to measure a difference between the in silico behavior of the reaction and the expected behavior of the reaction. The difference may be measured based on a comparison between a maximum velocity $V_{max}$ and a Michaelis constant $K_m$ of the in silico behavior of the reaction to the maximum velocity $V_{max}$ and the Michaelis constant $K_m$ of the expected behavior of the reaction.

At block 2825, updated forward and reverse rate constants are derived for each component step of the plurality of component steps representing the reaction based on the new energy profile. At block 2830, an updated in silico behavior of the reaction is derived based on the contribution of each component step of the plurality of component steps to the rate of change of the molecules within the reaction. The contribution of each component may be determined from the standard mathematical construct applying the updated forward and reverse rate constants to the one or more concentrations of molecules, respectively.

While the techniques for modeling kinetics are described herein at some length and with some particularity with respect to biochemical reactions including enzymes, substrates, and products, it is not intended that the techniques be limited to enzymatic reactions or any such particular enzymatic rate constants. Instead, it should be understood that the described techniques are provided as examples for parameterizing and modeling kinetics of reactions; the characteristics and parameters described with respect to the techniques for parameterizing and modeling kinetics of reactions are to be construed with the broadest sense to include variations of the particular characteristics and parameters. For example, the techniques may be used to determine kinetics of any chemical reaction, which may be used to additionally or alternatively constrain the model of a system.

## VII. **Example Computing Environment**

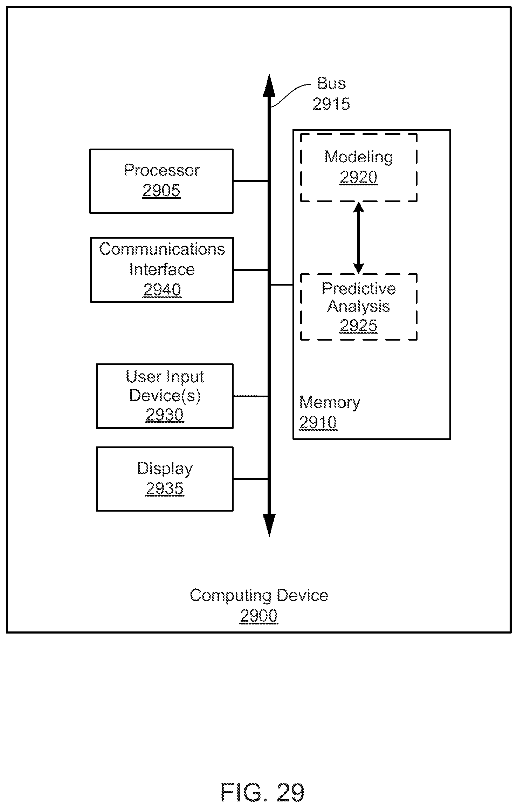

FIG. 29 illustrates an example computing device 2900 suitable for modeling in silico the kinetics of systems of connected biochemical reactions according to this disclosure (e.g., running a module of biological system model 200 described with respect to FIG. 2). The example computing device 2900 includes a processor 2905 which is in communication with the memory 2910 and other components of the computing device 2900 using one or more communications buses 2915. The processor 2905 is configured to execute processor-executable instructions stored in the memory 2910 to perform one or more methods for modeling in silico the kinetics of systems of connected biochemical reactions according to different examples, such as part or all of the example processes 400, 600, 2700, and 2800, described herein with respect to FIGS. 1-28. In this example, the memory 2910 stores processor-executable instructions that provide a modeling module 2920 and a predictive analysis module 2925 for one or more reaction pathways, processes, or systems of interest, as discussed herein with respect to FIGS. 1-28.

The modeling module 2920 (e.g., part of the biological system model 200 described with respect to FIG. 2) may be configured to derive a in silico behavior of the system based on the contribution of each component step of the plurality of component steps to the rate of change of the molecules within a pathway, process, or reaction. The predictive analysis module 2925 may be configured to predict a new energy profile, using a prediction model (e.g., prediction model 2410 described with respect to FIG. 24), for the reaction based on the derived in silico behavior of the reaction. Thereafter, the contribution of each component step to the rate of change of the molecules within the pathway, process, or reaction may be applied iteratively over a unit of time back to a state vector. For example, after results have been generated by the modeling module 2920 and the predictive analysis module 2925, a cross-module result synthesizor can access the module-specific results (from one or more module-specific results data stores or direct data availing) and synthesize the results to update high-level data such as a state vector (e.g., stored in a high-level metabolite data store). The state vector may then be used as input to simulate states and reactions of modules configured by an integrated development environment (e.g., the interaction system 100 described with respect to FIG. 1) and/or used by a simulator (e.g., module-specific simulation controller 500 described with respect to FIG. 5) to generate metabolite time-course data according to various embodiments.

The computing device 2900, in this example, also includes one or more user input devices 2930, such as a keyboard, mouse, touchscreen, microphone, etc., to accept user input. The computing device 2900 also includes a display 2935 to provide visual output to a user such as a user interface. The computing device 2900 also includes a communications interface 2940. In some examples, the communications interface 2940 may enable communications using one or more networks, including a local area network ("LAN"); wide area network ("WAN"), such as the Internet; metropolitan area network ("MAN"); point-to-point or peer-to-peer connection; etc. Communication with other devices (e.g., other devices within the interaction system 100 described with respect to FIG. 1) may be accomplished using any suitable networking protocol. For example, one suitable networking protocol may include the Internet Protocol ("IP"), Transmission Control Protocol ("TCP"), User Datagram Protocol ("UDP"), or combinations thereof, such as TCP/IP or UDP/IP.

## VIII. **Additional Considerations**

Specific details are given in the above description to provide a thorough understanding of the embodiments. However, it is understood that the embodiments can be practiced without these specific details. For example, circuits can be shown in block diagrams in order not to obscure the embodiments in unnecessary detail. In other instances, well-known circuits, processes, algorithms, structures, and techniques can be shown without unnecessary detail in order to avoid obscuring the embodiments.

Implementation of the techniques, blocks, steps and means described above can be done in various ways. For example, these techniques, blocks, steps and means can be implemented in hardware, software, or a combination thereof. For a hardware implementation, the processing units can be implemented within one or more application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described above, and/or a combination thereof.

Also, it is noted that the embodiments can be described as a process which is depicted as a flowchart, a flow diagram, a data flow diagram, a structure diagram, or a block diagram. Although a flowchart can describe the operations as a sequential process, many of the operations can be performed in parallel or concurrently. In addition, the order of the operations can be re-arranged. A process is terminated when its operations are completed, but could have additional steps not included in the figure. A process can correspond to a method, a function, a procedure, a subroutine, a subprogram, etc. When a process corresponds to a function, its termination corresponds to a return of the function to the calling function or the main function.

Furthermore, embodiments can be implemented by hardware, software, scripting languages, firmware, middleware, microcode, hardware description languages, and/or any combination thereof. When implemented in software, firmware, middleware, scripting language, and/or microcode, the program code or code segments to perform the necessary tasks can be stored in a machine readable medium such as a storage medium. A code segment or machine-executable instruction can represent a procedure, a function, a subprogram, a program, a routine, a subroutine, a module, a software package, a script, a class, or any combination of instructions, data structures, and/or program statements. A code segment can be coupled to another code segment or a hardware circuit by passing and/or receiving information, data, arguments, parameters, and/or memory contents. Information, arguments, parameters, data, etc. can be passed, forwarded, or transmitted via any suitable means including memory sharing, message passing, ticket passing, network transmission, etc.

For a firmware and/or software implementation, the methodologies can be implemented with modules (e.g., procedures, functions, and so on) that perform the functions described herein. Any machine-readable medium tangibly embodying instructions can be used in implementing the methodologies described herein. For example, software codes can be stored in a memory. Memory can be implemented within the processor or external to the processor. As used herein the term "memory" refers to any type of long term, short term, volatile, nonvolatile, or other storage medium and is not to be limited to any particular type of memory or number of memories, or type of media upon which memory is stored.

Moreover, as disclosed herein, the term "storage medium", "storage" or "memory" can represent one or more memories for storing data, including read only memory (ROM), random access memory (RAM), magnetic RAM, core memory, magnetic disk storage mediums, optical storage mediums, flash memory devices and/or other machine readable mediums for storing information. The term "machine-readable medium" includes, but is not limited to portable or fixed storage devices, optical storage devices, wireless channels, and/or various other storage mediums capable of storing that contain or carry instruction(s) and/or data.

While the principles of the disclosure have been described above in connection with specific apparatuses and methods, it is to be clearly understood that this description is made only by way of example and not as limitation on the scope of the disclosure.

## Claims

1. A system comprising:

   one or more data processors (2905); and
   a non-transitory computer readable storage medium (2910) containing instructions which, when executed on the one or more data processors (2905), cause the one or more data processors (2905) to perform actions including:

   obtaining (2805) an initial energy profile for a reaction, wherein the reaction is part of a pathway or process in a system to be modeled;
   deriving (2810) forward and reverse rate constants for each component step of a plurality of component steps representing the reaction based on the initial energy profile, wherein each component step is represented by a standard mathematical construct comprising a set of forward and reverse rate equations that apply the forward and reverse rate constants to one or more concentrations of molecules, respectively;
   deriving (2815) a *in silico* behavior of the reaction based on the contribution of each component step of the plurality of component steps to a rate of change of the molecules within the reaction, wherein the contribution of each component is determined from the standard mathematical construct applying the forward and reverse rate constants to the one or more concentrations of molecules, respectively;
   predicting (2820), using a machine learning prediction model trained to predict energy profiles for the reaction, a new energy profile for the reaction based on the derived *in silico* behavior of the reaction, wherein the predicting comprises learning a set of parameters including free energy states, which are changeable to define a path of the reaction from substrate to product to fit the *in silico* behavior of the reaction to an expected behavior of the reaction;

deriving (2825) updated forward and reverse rate constants for each component step of the plurality of component steps representing the reaction based on the new energy profile;

deriving (2830) an updated *in silico* behavior of the reaction based on the contribution of each component step of the plurality of component steps to the rate of change of the molecules within the reaction, wherein the contribution of each component is determined from the standard mathematical construct applying the updated forward and reverse rate constants to the one or more concentrations of molecules, respectively; and

transmitting an instruction for performing an experiment on an experimental system that corresponds to the *in silico* behavior of the reaction.

2. The system of claim 1, wherein the prediction model comprises a loss function constructed to measure a difference between the *in silico* behavior of the reaction and the expected behavior of the reaction, and wherein the difference is measured based on a comparison between a maximum velocity $V_{max}$ and a Michaelis constant $K_m$ of the *in silico* behavior of the reaction to the maximum velocity $V_{max}$ and the Michaelis constant $K_m$ of the expected behavior of the reaction.

3. The system of claim 1, wherein the plurality of component steps comprise at least two binding component steps and at least one transition component step, wherein each binding component step of the at least two binding component steps comprises: (i) binding between an enzyme and a substrate, or (ii) dissociation of an enzyme from a product, and wherein each transition component step of the at least one transition component step comprises a chemical conversion of an enzyme:substrate complex to a product:enzyme complex.

4. The system of claim 3, wherein the binding between the substrate and the enzyme contributes a first $\Delta G$ overall energy to the energy profile, the dissociation of the enzyme from the product contributes a second $\Delta G$ overall energy to the energy profile, and the chemical conversion of the enzyme: substrate complex to the product:enzyme complex contributes a $\Delta G\ddagger$ activation energy and a third $\Delta G$ overall energy to the energy profile.

5. The system of claim 1, wherein each forward rate equation has a form of a forward rate constant $k_{on}$ or $k_{fwd}$ multiplied by a single concentration or two concentrations, and wherein each reverse rate equation has a form of a reverse rate constant $k_{off}$ or $k_{rev}$ multiplied by a single concentration or two concentrations.

6. The system of claim 5, wherein the deriving the *in silico* behavior of the reaction comprises numerically integrating across the standard mathematical constructs using a system of ordinary differential equations to determine the contribution of each component step to the rate of change of molecules within the reaction.

7. The system of claim 6, wherein the system of ordinary differential equations is represented by a single matrix built piecewise indicating the contribution of each component step to the rate of change of the molecules within the reaction, and wherein the actions further comprise applying iteratively the contribution of each component step to the rate of change of the molecules within the reaction over a unit of time back to a state vector.

8. A computer-implemented method comprising:

obtaining (2805) an initial energy profile for a reaction, wherein the reaction is part of a pathway or process in a system to be modeled;

deriving (2810) forward and reverse rate constants for each component step of a plurality of component steps representing the reaction based on the initial energy profile, wherein each component step is represented by a standard mathematical construct comprising a set of forward and reverse rate equations that apply the forward and reverse rate constants to one or more concentrations of molecules, respectively;

deriving (2815) a *in silico* behavior of the reaction based on the contribution of each component step of the plurality of component steps to a rate of change of the molecules within the reaction, wherein the contribution of each component is determined from the standard mathematical construct applying the forward and reverse rate constants to the one or more concentrations of molecules, respectively;

predicting (2820), using a machine learning prediction model trained to predict energy profiles for the reaction, a new energy profile for the reaction based on the derived *in silico* behavior of the reaction, wherein the predicting comprises learning a set of parameters including free energy states, which are changeable to define a path of the reaction from substrate to product to fit the *in silico* behavior of the reaction to an expected behavior of the reaction;

deriving (2825) updated forward and reverse rate constants for each component step of the plurality of component steps representing the reaction based on the new energy profile;

deriving (2830) an updated *in silico* behavior of the reaction based on the contribution of each component step of the plurality of component steps to the rate of change of the molecules within the reaction, wherein the contribution of each component is determined from the standard mathematical construct applying the updated forward and reverse rate constants to the one or more concentrations of molecules, respectively; and

transmitting an instruction for performing an experiment on an experimental system that corresponds to the *in silico* behavior of the reaction.

9. The computer-implemented method of claim 8, wherein the prediction model comprises a loss function constructed to measure a difference between the *in silico* behavior of the reaction and the expected behavior of the reaction, and wherein the difference is measured based on a comparison between a maximum velocity $V_{max}$ and a Michaelis constant $K_m$ of the *in silico* behavior of the reaction to the maximum velocity $V_{max}$ and the Michaelis constant $K_m$ of the expected behavior of the reaction.

10. The computer-implemented method of claim 8, wherein the plurality of component steps comprise at least two binding component steps and at least one transition component step, wherein each binding component step of the at least two binding component steps comprises: (i) binding between an enzyme and a substrate, or (ii) dissociation of an enzyme from a product, and wherein each transition component step of the at least one transition component step comprises a chemical conversion of an enzyme: substrate complex to a product:enzyme complex.

11. The computer-implemented method of claim 10, wherein the binding between the substrate and the enzyme contributes a first ΔG overall energy to the energy profile, the dissociation of the enzyme from the product contributes a second ΔG overall energy to the energy profile, and the chemical conversion of the enzyme: substrate complex to the product:enzyme complex contributes a ΔG‡ activation energy and a third ΔG overall energy to the energy profile.

12. The computer-implemented method of claim 8, wherein each forward rate equation has a form of a forward rate constant $k_{on}$ or $k_{fwd}$ multiplied by a single concentration or two concentrations, and wherein each reverse rate equation has a form of a reverse rate constant $k_{off}$ or $k_{rev}$ multiplied by a single concentration or two concentrations.

13. The computer-implemented method of claim 12, wherein the deriving the *in silico* behavior of the reaction comprises numerically integrating across the standard mathematical constructs using a system of ordinary differential equations to determine the contribution of each component step to the rate of change of molecules within the reaction.

14. The computer-implemented method of claim 13, wherein the system of ordinary differential equations is represented by a single matrix built piecewise indicating the contribution of each component step to the rate of change of the molecules within the reaction.

15. A computer-program product tangibly embodied in a non-transitory machine-readable storage medium (2910), including instructions configured to cause one or more data processors (2905) to perform the computer-implemented method of claims 8 to 14.

**Patentansprüche**

1. System, umfassend:

einen oder mehrere Datenprozessoren (2905); und

ein nichtflüchtiges computerlesbares Speichermedium (2910), das Anweisungen enthält, die, wenn sie auf dem einen oder den mehreren Datenprozessoren (2905) ausgeführt werden, den einen oder die mehreren Datenprozessoren (2905) veranlassen, Aktionen durchzuführen, die Folgendes einschließen:

Erhalten (2805) eines Anfangsenergieprofils für eine Reaktion, wobei die Reaktion Teil eines Pfads oder Prozesses in einem zu modellierenden System ist;

Ableiten (2810) einer Vorwärts- und einer Rückwärts-Geschwindigkeitskonstante für jeden Komponentenschritt einer Vielzahl von Komponentenschritten, die die Reaktion darstellen, basierend auf dem Anfangsenergieprofil, wobei jeder Komponentenschritt durch ein mathematisches Standardkonstrukt dargestellt wird, das einen Satz aus einer Vorwärts- und einer Rückwärts-Geschwindigkeitsgleichung umfasst, die die Vorwärts- und die Rückwärts-Geschwindigkeitskonstante auf jeweils eine oder mehrere Konzentrationen von Molekülen anwenden;

Ableiten (2815) eines In-silico-Verhaltens der Reaktion basierend auf dem Beitrag jedes Komponentenschritts der Vielzahl von Komponentenschritten zu einer Änderungsrate der Moleküle innerhalb der Reaktion, wobei der Beitrag jeder Komponente aus dem mathematischen Standardkonstrukt bestimmt wird, das die Vorwärts- und die Rückwärts-Geschwindigkeitskonstante auf die jeweils eine oder die mehreren Konzentrationen von Molekülen anwendet;

Vorhersagen (2820), unter Verwendung eines Vorhersagemodells für maschinelles Lernen, das trainiert ist, um Energieprofile für die Reaktion vorherzusagen, eines neuen Energieprofils für die Reaktion basierend auf dem abgeleiteten *In-silico-Verhalten* der Reaktion, wobei das Vorhersagen ein Lernen eines Satzes von Parametern einschließlich Zuständen freier Energie umfasst, die änderbar sind, um einen Pfad der Reaktion von Substrat zu Produkt zu definieren, um das *In-silico-Verhalten* der Reaktion an ein erwartetes Verhalten der Reaktion anzupassen;

Ableiten (2825) einer aktualisierten Vorwärts- und einer aktualisierten Rückwärts-Geschwindigkeitskonstante für jeden Komponentenschritt der Vielzahl von Komponentenschritten, die die Reaktion darstellen, basierend auf dem neuen Energieprofil;

Ableiten (2830) eines aktualisierten In-silico-Verhaltens der Reaktion basierend auf dem Beitrag jedes Komponentenschritts der Vielzahl von Komponentenschritten zu der Änderungsrate der Moleküle innerhalb der Reaktion, wobei der Beitrag jeder Komponente aus dem mathematischen Standardkonstrukt bestimmt wird, das die eine aktualisierte Vorwärts- und die eine aktualisierte Rückwärts-Geschwindigkeitskonstante auf die jeweils eine oder die mehreren Konzentrationen von Molekülen anwendet; und

Übertragen einer Anweisung zum Durchführen eines Experiments an einem experimentellen System, das dem *In-silico-Verhalten* der Reaktion entspricht.

2. System nach Anspruch 1, wobei das Vorhersagemodell eine Verlustfunktion umfasst, die konfiguriert ist, um eine Differenz zwischen dem *In-silico-Verhalten* der Reaktion und dem erwarteten Verhalten der Reaktion zu messen, und wobei die Differenz basierend auf einem Vergleich zwischen einer maximalen Geschwindigkeit $V_{max}$ und einer Michaelis-Konstante $K_m$ des *In*-silico-Verhaltens der Reaktion mit der maximalen Geschwindigkeit $V_{max}$ und der Michaelis-Konstante $K_m$ des erwarteten Verhaltens der Reaktion gemessen wird.

3. System nach Anspruch 1, wobei die Vielzahl von Komponentenschritten mindestens zwei Bindungskomponentenschritte und mindestens einen Übergangskomponentenschritt umfasst, wobei jeder Bindungskomponentenschritt der mindestens zwei Bindungskomponentenschritte Folgendes umfasst: (i) Bindung zwischen einem Enzym und einem Substrat oder (ii) Dissoziation eines Enzyms von einem Produkt, und wobei jeder Übergangskomponentenschritt des mindestens einen Übergangskomponentenschritts eine chemische Umwandlung eines Enzym: Substrat-Komplexes in einen Produkt:Enzym-Komplex umfasst.

4. System nach Anspruch 3, wobei die Bindung zwischen dem Substrat und dem Enzym eine erste ∆G-Gesamtenergie zu dem Energieprofil beiträgt, die Dissoziation des Enzyms von dem Produkt eine zweite ∆G-Gesamtenergie zu dem Energieprofil beiträgt und die chemische Umwandlung des Enzym: Substrat-Komplexes in den Produkt:Enzym-Komplex eine ∆G‡-Aktivierungsenergie und eine dritte ∆G-Gesamtenergie zu dem Energieprofil beiträgt.

5. System nach Anspruch 1, wobei jede Vorwärts-Geschwindigkeitsgleichung eine Form einer Vorwärts-Geschwindigkeitskonstante $k_{on}$ oder $k_{fwd}$ multipliziert mit einer einzelnen Konzentration oder zwei Konzentrationen aufweist, und wobei jede Rückwärts-Geschwindigkeitsgleichung eine Form einer Rückwärts-Geschwindigkeitskonstante $k_{off}$ oder $k_{rev}$ multipliziert mit einer einzelnen Konzentration oder zwei Konzentrationen aufweist.

6. System nach Anspruch 5, wobei das Ableiten des *In-silico*-Verhaltens der Reaktion ein numerisches Integrieren über die mathematischen Standardkonstrukte unter Verwendung eines Systems gewöhnlicher Differentialgleichungen umfasst, um den Beitrag jedes Komponentenschritts zu der Änderungsrate von Molekülen innerhalb der Reaktion zu bestimmen.

7. System nach Anspruch 6, wobei das System gewöhnlicher Differentialgleichungen durch eine einzelne, stückweise aufgebaute Matrix dargestellt wird, die den Beitrag jedes Komponentenschritts zu der Änderungsrate der Moleküle innerhalb der Reaktion angibt, und wobei die Aktionen ferner ein iteratives Anwenden des Beitrags jedes Komponentenschritts zu der Änderungsrate der Moleküle innerhalb der Reaktion über eine Zeiteinheit zurück auf einen Zustandsvektor umfassen.

8. Computerimplementiertes Verfahren, umfassend:

Erhalten (2805) eines Anfangsenergieprofils für eine Reaktion, wobei die Reaktion Teil eines Pfads oder Prozesses in einem zu modellierenden System ist;

Ableiten (2810) einer Vorwärts- und einer Rückwärts-Geschwindigkeitskonstante für jeden Komponentenschritt einer Vielzahl von Komponentenschritten, die die Reaktion darstellen, basierend auf dem Anfangsenergieprofil, wobei jeder Komponentenschritt durch ein mathematisches Standardkonstrukt dargestellt wird, das einen Satz aus einer Vorwärts- und einer Rückwärts-Geschwindigkeitsgleichung umfasst, die die Vorwärts- und die Rückwärts-Geschwindigkeitskonstante auf jeweils eine oder mehrere Konzentrationen von Molekülen anwenden;

Ableiten (2815) eines In-silico-Verhaltens der Reaktion basierend auf dem Beitrag jedes Komponentenschritts der Vielzahl von Komponentenschritten zu einer Änderungsrate der Moleküle innerhalb der Reaktion, wobei der Beitrag jeder Komponente aus dem mathematischen Standardkonstrukt bestimmt wird, das die Vorwärts- und die Rückwärts-Geschwindigkeitskonstante auf die jeweils eine oder die mehreren Konzentrationen von Molekülen anwendet;

Vorhersagen (2820), unter Verwendung eines Vorhersagemodells für maschinelles Lernen, das trainiert ist, um Energieprofile für die Reaktion vorherzusagen, eines neuen Energieprofils für die Reaktion basierend auf dem abgeleiteten *In-silico-Verhalten* der Reaktion, wobei das Vorhersagen ein Lernen eines Satzes von Parametern einschließlich Zuständen freier Energie umfasst, die änderbar sind, um einen Pfad der Reaktion von Substrat zu Produkt zu definieren, um das *In-silico-Verhalten* der Reaktion an ein erwartetes Verhalten der Reaktion anzupassen;

Ableiten (2825) einer aktualisierten Vorwärts- und einer aktualisierten Rückwärts-Geschwindigkeitskonstante für jeden Komponentenschritt der Vielzahl von Komponentenschritten, die die Reaktion darstellen, basierend auf dem neuen Energieprofil;

Ableiten (2830) eines aktualisierten In-silico-Verhaltens der Reaktion basierend auf dem Beitrag jedes Komponentenschritts der Vielzahl von Komponentenschritten zu der Änderungsrate der Moleküle innerhalb der Reaktion, wobei der Beitrag jeder Komponente aus dem mathematischen Standardkonstrukt bestimmt wird, das die eine aktualisierte Vorwärts- und die eine aktualisierte Rückwärts-Geschwindigkeitskonstante auf die jeweils eine oder die mehreren Konzentrationen von Molekülen anwendet; und

Übertragen einer Anweisung zum Durchführen eines Experiments an einem experimentellen System, das dem *In-silico-Verhalten* der Reaktion entspricht.

9. Computerimplementiertes Verfahren nach Anspruch 8, wobei das Vorhersagemodell eine Verlustfunktion umfasst, die konfiguriert ist, um eine Differenz zwischen dem *In-silico-Verhalten* der Reaktion und dem erwarteten Verhalten der Reaktion zu messen, und wobei die Differenz basierend auf einem Vergleich zwischen einer maximalen Geschwindigkeit $V_{max}$ und einer Michaelis-Konstante $K_m$ des In-silico-Verhaltens der Reaktion mit der maximalen Geschwindigkeit $V_{max}$ und der Michaelis-Konstante $K_m$ des erwarteten Verhaltens der Reaktion gemessen wird.

10. Computerimplementiertes Verfahren nach Anspruch 8, wobei die Vielzahl von Komponentenschritten mindestens zwei Bindungskomponentenschritte und mindestens einen Übergangskomponentenschritt umfasst, wobei jeder Bindungskomponentenschritt der mindestens zwei Bindungskomponentenschritte Folgendes umfasst: (i) Bindung zwischen einem Enzym und einem Substrat oder (ii) Dissoziation eines Enzyms von einem Produkt, und wobei jeder Übergangskomponentenschritt des mindestens einen Übergangskomponentenschritts eine chemische Umwandlung eines Enzym: Substrat-Komplexes in einen Produkt:Enzym-Komplex umfasst.

11. Computerimplementiertes Verfahren nach Anspruch 10, wobei die Bindung zwischen dem Substrat und dem Enzym eine erste ΔG - Gesamtenergie zu dem Energieprofil beiträgt, die Dissoziation des Enzyms von dem Produkt eine zweite ΔG -Gesamtenergie zu dem Energieprofil beiträgt und die chemische Umwandlung des Enzym:Substrat-Komplexes in den Produkt:Enzym-Komplex eine ΔG‡ -Aktivierungsenergie und eine dritte ΔG -Gesamtenergie zu dem Energieprofil beiträgt.

12. Computerimplementiertes Verfahren nach Anspruch 8, wobei jede Vorwärts-Geschwindigkeitsgleichung eine Form einer Vorwärts-Geschwindigkeitskonstante $k_{on}$ oder $k_{fwd}$ multipliziert mit einer einzelnen Konzentration oder zwei Konzentrationen aufweist, und wobei jede Rückwärts-Geschwindigkeitsgleichung eine Form einer Rückwärts-Geschwindigkeitskonstante $k_{off}$ oder $k_{rev}$ multipliziert mit einer einzelnen Konzentration oder zwei Konzentrationen aufweist.

13. Computerimplementiertes Verfahren nach Anspruch 12, wobei das Ableiten des In-silico-Verhaltens der Reaktion ein numerisches Integrieren über die mathematischen Standardkonstrukte unter Verwendung eines Systems gewöhnlicher Differentialgleichungen umfasst, um den Beitrag jedes Komponentenschritts zu der Änderungsrate von Molekülen innerhalb der Reaktion zu bestimmen.

14. Computerimplementiertes Verfahren nach Anspruch 13, wobei das System gewöhnlicher Differentialgleichungen durch eine einzelne, stückweise aufgebaute Matrix dargestellt wird, die den Beitrag jedes Komponentenschritts zu der Änderungsrate der Moleküle innerhalb der Reaktion angibt.

15. Computerprogrammprodukt, das greifbar in einem nichtflüchtigen maschinenlesbaren Speichermedium (2910) verkörpert ist, einschließlich Anweisungen, die konfiguriert sind, um einen oder mehrere Datenprozessoren (2905) zu veranlassen, das computerimplementierte Verfahren nach den Ansprüchen 8 bis 14 durchzuführen.

**Revendications**

1. Système comprenant :

un ou plusieurs processeurs de données (2905) ; et
un support de stockage non transitoire lisible par ordinateur (2910) contenant des instructions qui, lorsqu'elles sont exécutées par les un ou plusieurs processeurs de données (2905), amènent les un ou plusieurs processeurs de données (2905) à réaliser des actions comportant :

l'obtention (2805) d'un profil énergétique initial pour une réaction, dans lequel la réaction fait partie d'une voie ou d'un processus dans un système à modéliser ;
la dérivation (2810) des constantes de vitesse directe et inverse pour chaque étape composante d'une pluralité d'étapes composantes représentant la réaction basée sur le profil énergétique initial, dans lequel chaque étape composante est représentée par une construction mathématique standard comprenant un ensemble d'équations de vitesse directe et inverse qui appliquent les constantes de vitesse directe et inverse à une ou plusieurs concentrations de molécules, respectivement ;
la dérivation (2815) d'un comportement *in silico* de la réaction sur la base de la contribution de chaque étape composante de la pluralité d'étapes composantes à un taux de changement des molécules au sein de la réaction, dans lequel la contribution de chaque composante est déterminée à partir de la construction mathématique standard appliquant les constantes de vitesse directe et inverse aux une ou plusieurs concentrations de molécules, respectivement ;
la prédiction (2820), à l'aide d'un modèle de prédiction d'apprentissage automatique entraîné à prédire les profils énergétiques de la réaction, d'un nouveau profil énergétique pour la réaction sur la base du comportement *in silico* dérivé de la réaction, dans lequel la prédiction comprend l'apprentissage d'un ensemble de paramètres comportant les états d'énergie libre, qui sont modifiables pour définir un chemin de la réaction du substrat au produit afin d'adapter le comportement *in silico* de la réaction à un comportement attendu de la réaction ;
la dérivation (2825) des constantes de vitesse directe et inverse mises à jour pour chaque étape composante de la pluralité d'étapes composantes représentant la réaction sur la base du nouveau profil énergétique ;
la dérivation (2830) d'un comportement *in silico* mis à jour de la réaction sur la base de la contribution de chaque étape composante de la pluralité d'étapes composantes au taux de changement des molécules au sein de la réaction, dans lequel la contribution de chaque composante est déterminée à partir de la construction mathématique standard appliquant les constantes de vitesse directe et inverse mises à jour aux une ou plusieurs concentrations de molécules, respectivement ; et
la transmission d'une instruction pour réaliser une expérience sur un système expérimental qui correspond au comportement *in silico* de la réaction.

2. Système selon la revendication 1, dans lequel le modèle de prédiction comprend une fonction de perte construite pour mesurer une différence entre le comportement *in silico* de la réaction et le comportement attendu de la réaction, et dans lequel la différence est mesurée sur la base d'une comparaison entre une vitesse maximale $V_{max}$ et une constante de Michaelis $K_m$ du comportement *in silico* de la réaction à la vitesse maximale $V_{max}$ et à la constante de Michaelis $K_m$ du comportement attendu de la réaction.

3. Système selon la revendication 1, dans lequel la pluralité d'étapes composantes comprend au moins deux étapes composantes de liaison et au moins une étape composante de transition, dans lequel chaque étape composante de liaison des au moins deux étapes composantes de liaison comprennent : (i) la liaison entre une enzyme et un substrat, ou (ii) la dissociation d'une enzyme d'un produit, et dans lequel chaque étape composante de transition de l'au moins une étape composante de transition comprend une conversion chimique d'un complexe enzyme:substrat en un complexe produit:enzyme.

**4.** Système selon la revendication 3, dans lequel la liaison entre le substrat et l'enzyme apporte une première énergie globale ΔG au profil énergétique, la dissociation de l'enzyme du produit apporte une deuxième énergie globale ΔG au profil énergétique, et la conversion chimique du complexe enzyme:substrat en complexe produit:enzyme apporte une énergie d'activation ΔG‡ et une troisième énergie globale ΔG au profil énergétique.

**5.** Système selon la revendication 1, dans lequel chaque équation de vitesse directe a une forme d'une constante de vitesse directe $k_{on}$ ou $k_{fwd}$ multipliée par une seule concentration ou deux concentrations, et dans lequel chaque équation de vitesse inverse a une forme d'une constante de vitesse inverse $k_{off}$ ou $k_{rev}$ multipliée par une seule concentration ou deux concentrations.

**6.** Système selon la revendication 5, dans lequel la dérivation du comportement *in silico* de la réaction comprend l'intégration numérique des constructions mathématiques standard à l'aide d'un système d'équations différentielles ordinaires pour déterminer la contribution de chaque étape composante au taux de changement des molécules au sein de la réaction.

**7.** Système selon la revendication 6, dans lequel le système d'équations différentielles ordinaires est représenté par une matrice unique construite par morceaux indiquant la contribution de chaque étape composante au taux de changement des molécules au sein de la réaction, et dans lequel les actions comprennent en outre l'application itérative de la contribution de chaque étape composante au taux de changement des molécules au sein de la réaction sur une unité de temps à un vecteur d'état.

**8.** Procédé mis en œuvre par ordinateur comprenant :

l'obtention (2805) d'un profil énergétique initial pour une réaction, dans lequel la réaction fait partie d'une voie ou d'un processus dans un système à modéliser ;

la dérivation (2810) des constantes de vitesse directe et inverse pour chaque étape composante d'une pluralité d'étapes composantes représentant la réaction basée sur le profil énergétique initial, dans lequel chaque étape composante est représentée par une construction mathématique standard comprenant un ensemble d'équations de vitesse directe et inverse qui appliquent les constantes de vitesse directe et inverse à une ou plusieurs concentrations de molécules, respectivement ;

la dérivation (2815) d'un comportement in *silico* de la réaction sur la base de la contribution de chaque étape composante de la pluralité d'étapes composantes à un taux de changement des molécules au sein de la réaction, dans lequel la contribution de chaque composante est déterminée à partir de la construction mathématique standard appliquant les constantes de vitesse directe et inverse aux une ou plusieurs concentrations de molécules, respectivement ;

la prédiction (2820), à l'aide d'un modèle de prédiction d'apprentissage automatique entraîné à prédire les profils énergétiques de la réaction, d'un nouveau profil énergétique pour la réaction sur la base du comportement in *silico dérivé de la réaction, dans* lequel la prédiction comprend l'apprentissage d'un ensemble de paramètres comportant les états d'énergie libre, qui sont modifiables pour définir un chemin de la réaction du substrat *au produit afin d'adapter le comportement in silico* de la réaction à un comportement attendu de la réaction ;

la dérivation (2825) des constantes de vitesse directe et inverse mises à jour pour chaque étape composante de la pluralité d'étapes composantes représentant la réaction sur la base du nouveau profil énergétique ;

la dérivation (2830) d'un comportement in *silico* mis à jour de la réaction sur la base de la contribution de chaque étape composante de la pluralité d'étapes composantes au taux de changement des molécules au sein de la réaction, dans lequel la contribution de chaque composante est déterminée à partir de la construction mathématique standard appliquant les constantes de vitesse directe et inverse mises à jour aux une ou plusieurs concentrations de molécules, respectivement ; et

la transmission d'une instruction pour réaliser une expérience sur un système expérimental qui correspond au comportement *in silico* de la réaction.

**9.** Procédé mis en œuvre par ordinateur selon la revendication 8, dans lequel le modèle de prédiction comprend une fonction de perte construite pour mesurer une différence entre le comportement *in silico* de la réaction et le comportement attendu de la réaction, et dans lequel la différence est mesurée sur la base d'une comparaison entre une vitesse maximale $V_{max}$ et une constante de Michaelis $K_m$ du comportement *in silico* de la réaction à la vitesse

maximale $V_{max}$ et à la constante de Michaelis $K_m$ du comportement attendu de la réaction.

10. Procédé mis en œuvre par ordinateur selon la revendication 8, dans lequel la pluralité d'étapes composantes comprend au moins deux étapes composantes de liaison et au moins une étape composante de transition, dans lequel chaque étape composante de liaison des au moins deux étapes composantes de liaison comprennent : (i) la liaison entre une enzyme et un substrat, ou (ii) la dissociation d'une enzyme d'un produit, et dans lequel chaque étape composante de transition de l'au moins une étape composante de transition comprend une conversion chimique d'un complexe enzyme:substrat en un complexe produit:enzyme.

11. Procédé mis en œuvre par ordinateur selon la revendication 10, dans lequel la liaison entre le substrat et l'enzyme apporte une première énergie globale $\Delta G$ au profil énergétique, la dissociation de l'enzyme du produit apporte une deuxième énergie globale $\Delta G$ au profil énergétique, et la conversion chimique du complexe enzyme:substrat en complexe produit:enzyme apporte une énergie d'activation $\Delta G\ddagger$ et une troisième énergie globale $\Delta G$ au profil énergétique.

12. Procédé mis en œuvre par ordinateur selon la revendication 8, dans lequel chaque équation de vitesse directe a une forme d'une constante de vitesse directe $k_{on}$ ou $k_{fwd}$ multipliée par une seule concentration ou deux concentrations, et dans lequel chaque équation de vitesse inverse a la forme d'une constante de vitesse inverse $k_{off}$ ou $k_{rev}$ multipliée par une seule concentration ou deux concentrations.

13. Procédé mis en œuvre par ordinateur selon la revendication 12, dans lequel la dérivation du comportement *in silico* de la réaction comprend l'intégration numérique des constructions mathématiques standard à l'aide d'un système d'équations différentielles ordinaires pour déterminer la contribution de chaque étape composante au taux de changement des molécules au sein de la réaction.

14. Procédé mis en œuvre par ordinateur selon la revendication 13, dans lequel le système d'équations différentielles ordinaires est représenté par une seule matrice construite par morceaux indiquant la contribution de chaque étape composante au taux de changement des molécules au sein de la réaction.

15. Produit de programme informatique incorporé de manière tangible dans un support de stockage non transitoire lisible par machine (2910), comportant des instructions configurées pour amener un ou plusieurs processeurs de données (2905) à réaliser le procédé mis en œuvre par ordinateur selon les revendications 8 à 14.

100

FIG. 1

FIG. 2

FIG. 3

FIG. 4

FIG. 5

FIG. 6

FIG. 7

FIG. 8

FIG. 9

1005

1010

A

B

A:B

A          B

FIG. 10

1105

F6P          F6P

PGI

1110          1120          1125          1130

G6P          F6P

PGI:F6P          PGI:F6P

PGI          PGI

1115

FIG. 11

FIG. 12

FIG. 13

FIG. 14

FIG. 15

FIG. 16A

FIG. 16B

FIG. 16C

FIG. 16D

FIG. 16E

1710

A
B
A:B

1720

A ⟷ B

1705

$$v_{on} = k_{on} \cdot [A] \cdot [B]$$

$$v_{off} = k_{off} \cdot [A:B]$$

1715

$$v_{fwd} = k_{fwd} \cdot [A]$$

$$v_{rev} = k_{rev} \cdot [B]$$

$$d[A]/dt = -v_{on} + v_{off}$$

$$d[B]/dt = -v_{on} + v_{off}$$

$$d[A:B]/dt = v_{on} - v_{off}$$

$$d[A]/dt = -v_{fwd} + v_{rev}$$

$$d[B]/dt = v_{fwd} - v_{rev}$$

FIG. 17

1800

1805

Rate constants —— 1815

1820                1810                    1830                    1825

State ----> ODEs ----> Rates per component ----> d[state]/dt

FIG. 18A

Rate constants

1820                1810                                            1825

State ----> ODEs ----> Rates per component ----> d[state]/dt

· dt

1835

FIG. 18B

FIG. 18C

FIG. 18D

FIG. 19

EP 4 169 021 B1

2000

FIG. 20

2100

FIG. 21

FIG. 22

FIG. 23

FIG. 24

EP 4 169 021 B1

FIG. 25

**FIG. 26A**

**FIG. 26B**

2700

2705

Deconstruct a reaction into individual components

2710

Translate the individual components into standard
mathematical constructs

2715

Input forward and reverse rate constants into a system
of ordinary differential equations

2720

Input a state vector into the system of ordinary
differential equations

2725

Numerically integrated, using the system of ordinary
differential equations, to determine a contribution of each
component step to a rate of change of molecules within the
pathway or process

2730

Apply iteratively contribution of each component step to the
rate of change of molecules over a unit of time back to
the state vector

FIG. 27

2800

2805
Obtain initial energy profile for a reaction

2810
Derive forward and reverse rate constants for each
component step of the reaction based on the energy profile

2815
Derive *in silico* behavior of the reaction based on the
contribution of each component step to a rate of change
of the molecules within the reaction

2820
Predict a new energy profile, using a prediction model, for the
reaction based on the derived *in silico* behavior of the reaction

2825
Derive updated forward and reverse rate constants for each
component step of the reaction based on the energy profile

2830
Derive updated *in silico* behavior of the reaction based on the
contribution of each component step to the rate of change
of the molecules within the reaction

FIG. 28

FIG. 29

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DEMIREL YASAR**. Nonequilibrium thermodynamics modeling of coupled biochemical cycles in living cells. *Journal of non-Newtonian fluid mechanics*, 01 January 2010, 953-972